# EUROPEAN PATENT APPLICATION

(11) **EP 3 932 935 A1**
(43) Date of publication of application: **05.01.2022**
(21) Application number: 20305722.9
(22) Date of filing: 29.06.2020
(51) Int. Cl.: C07K 7/06, A61K 38/00, C07K 14/195

(54) **ANTIBACTERIAL PEPTIDES**

(71) Applicant: Centre National de la Recherche Scientifique, 75016 Paris (FR); Université de Strasbourg, 67000 Strasbourg (FR); Université de Bordeaux, 33000 Bordeaux (FR); Institut Polytechnique de Bordeaux, 33400 Talence (FR)
(72) Inventor: BURNOUF, Dominique, 67170 BRUMATH (FR); WAGNER, Jerôme, 67100 STRASBOURG (FR); COMPAIN, Guillaume, 33700 MERIGNAC (FR); GUICHARD, Gilles, 33170 GRADIGNAN (FR); CHRISTOPHE, André, 12310 Laissac (FR)
(74) Representative: Lavoix

(57) **Abstract**

The present invention relates to new peptides, in particular as antibacterial agents, and also to therapeutic uses thereof. The present invention also relates to said compounds for use for the treatment of bacterial infections.

This invention relates to the development of antibacterial peptides targeting the processivity factor of the bacterial replicative complex.

## Description

The present invention concerns new peptides, in particular as antibacterial agents, and also to therapeutic uses thereof. The present invention also relates to said compounds for use for the treatment of bacterial infections.

This invention relates to the development of antibacterial peptides targeting the processivity factor of the bacterial replicative complex.

The dramatic rise of antimicrobial resistance (AMR) in the recent years has finally caught the attention of governments and international organizations. Because it is a direct threat for human health, with a high cost economic impact, WHO and European Commission are now urging for a change in agricultural and medicinal practices and for the development of new antibiotics, in particular for the treatment of Gram negative (Gram -) infections. A first step toward this goal is to identify new bacterial molecular targets and to develop efficient molecules that will block their physiological functions. The bacterial processivity factor, also referred to as the sliding clamp (SC), is now identified as a potential drugable new target (P. Wolff et al., Journal of Medicinal Chemistry, 2011, 54, 4627-4637; A. S. Altieri and Z. Kelman, Front Mol Biosci, 2018, 5, 87; A. Kling et al., Science, 2015, 348, 1106-1112) .

Replicative DNA polymerase interact with SC and, as a consequence, become highly processive. Moreover, SC also serve as a molecular hub on which all the other DNA polymerases bind (poll, II, IV and V in E. coli) as well as other enzymes involved in DNA metabolism and interaction with SC is required for these proteins to fulfill their functions (O. J. Becherel, R. P. Fuchs and J. Wagner, DNA repair, 2002, 1, 703-708). Remarkably, in all cases, SC-protein primary interaction is mediated by a short peptide segment which encompasses the consensus sequence (QL[S/D]LF) (B. P. Dalrymple et al., Proc. Natl. Acad. Sci. U.S.A., 2001, 98, 11627-11632.).

Short synthetic peptides were previously developed and optimized to bind to *Escherichia coli* SC (*^{Ec}*SC) with an increased affinity as compared to natural peptides (P. Wolff et al., Journal of Medicinal Chemistry, 2011, 54, 4627-4637). These peptides also interact efficiently with SC from other Gram negative (Gram-) bacteria such as *Pseudomonas aeruginosa* (P. Wolff et al. Journal of Medicinal Chemistry, 2014, 57, 7565-7576), due to the high conservation of the pocket sequence among Gram- bacteria.

The aim of the present invention is thus to provide an efficient antimicrobial compound, in particular being able to bind the sliding claim with high affinity.

The aim of the present invention is also to provide a new class of antibiotics.

Therefore, the present invention relates to a compound of formula (I): 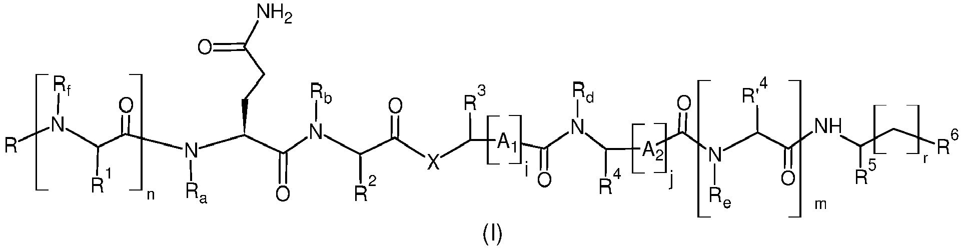 wherein:
- n is 0 or 1;
- m is 0 or 1;
- i is 0 or 1;
- j is 0 or 1;
- r is 0, 1 or 2;
- R is selected in the group consisting of:
   * a (C₁-C₁₂)alkyl group optionally substituted by a (C₆-C₁₀)aryl group, by a heteroaryl group or by a heterocycloalkyl group,
   * a (C₂-Ci₂)alkenyl group optionally substituted by a (C₆-C₁₀)aryl group,
   * a (C₃-C₆)cycloalkyl group,
   * a (C₆-C₁₀)aryl group optionally substituted by a (C₁-C₄)alkyl,
   * a group having the following formula (II): 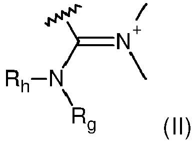 R_{g} and Rₕ representing independently from each other a (C₁-C₆)alkyl group, such as methyl, or forming together with the nitrogen atom carrying them a heterocycloalkyl group such as a morpholinyl group,
   * a group -C(=O)-R', R' being selected in the group consisting of:
      - a (C₁-C₁₂)alkyl group optionally substituted by an optionally substituted (C₆-C₁₀)aryl group,
      - a (C₂-Ci₂)alkenyl group optionally substituted by an optionally substituted C₆₋₁₀-aryl group or by a heteroaryl group,
      - a -NH-optionally substituted (C₆-C₁₀)aryl group,
      - a (C₃-C₆)cycloalkyl group,
      - a (C₆-C₁₀)aryl group optionally substituted by a (C₁-C₄)alkyl,
   * a group having the following formula (II-1): 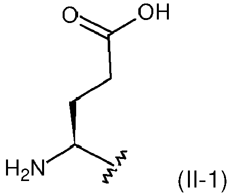
- X is O or a group N-R_{c};
- Rₐ, R_{b}, R_{c}, R_{d}, Rₑ, and R_{f} are, independently from each other, selected from the group consisting of: H, -CH₂-CH=CH₂, (C₁-C₆)alkyl groups, (C₆-C₁₀)aryl groups, and aralkyl groups;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
   or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
   * q is 1, 2, 3 or 4,
   * R^{7a} is a hydrogen atom, a (C₁-C₈)alkyl group, or an aralkyl group;
   * R^{7b} is a hydrogen atom, a (C₁-C₈)alkyl group, or an aralkyl group,
   * R⁸ is a hydrogen atom, a (C₁-C₈)alkyl group, or an aralkyl group, and
   * R⁹ is H or COR¹¹, R¹¹ being H or a (C₁-C₈)alkyl group, such as CH₃;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, such as being Me, and R'¹² being selected from the group consisting of: H, (C₁-C₈)alkyl group such as Me, and -C(=O)-(C₁-C₈)alkyl group, such as -C(=O)-CH₃;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)_{S}-, s being 1, 2, 3 or 4;
- R'⁴ is H, a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
   or R'⁴ forms a heterocycloalkyl group with Rₑ, the carbon atom carrying it and the nitrogen atom carrying Rₑ, such as for example a pyrrolidine or a piperidine or octahydroindole ring, said heterocycloalkyl group being optionally substituted with at least one hydroxyl group, amino group, or sulfur atom;
- R⁵ is selected in the group consisting of:
   * a -(CH₂)-(C₃-C₆)cycloalkyl group;
   * a -(CH₂-CH₂)-(C₃-C₆)cycloalkyl group;
   * a -(CH₂)-(C₆-C₁₀)aryl group optionally substituted by at least one halogen, (C₁-C₂)alkyl group and/or (C₁-C₂)alkoxy group;
   * a -(CH₂-CH₂)-(C₆-C₁₀)aryl group optionally substituted by at least one halogen, (C₁-C₂)alkyl group and/or (C₁-C₂)alkoxy group;
   * a -(CH₂)-(C₅-C₁₀)heteroaryl group optionally substituted by at least one halogen and/or (C₁-C₂)alkyl group;
   * a -(CH₂-CH₂)-(C₅-C₁₀)heteroaryl group optionally substituted by at least one halogen and/or (C₁-C₂)alkyl group; and
   * a (C₁-C₈)alkyl group or a (C₁-C₄)alkyl group optionally substituted by at least one halogen such as fluoroleucine;
- R⁶ is H, -CO₂H, -CO₂R¹⁰, -CO-NH₂, -CO-NHR₁₀, -OR¹⁰, -OH or NH₂ when r is 0 or 1 or 2, -NH-CO-NHR¹⁰ when r is 1 or 2; wherein
   * R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group;
the following compounds being excluded:

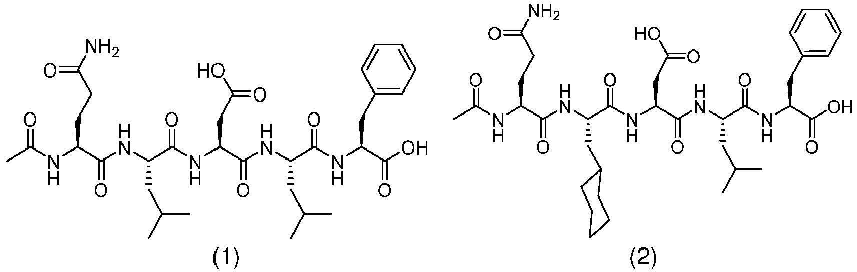

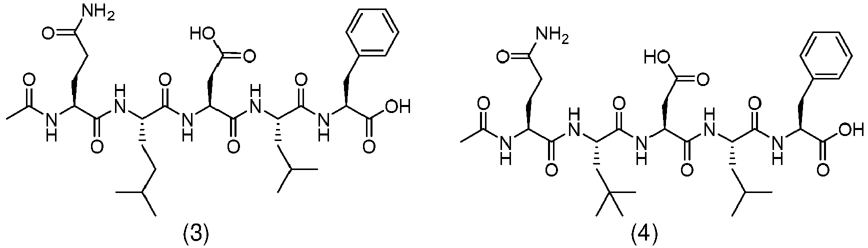

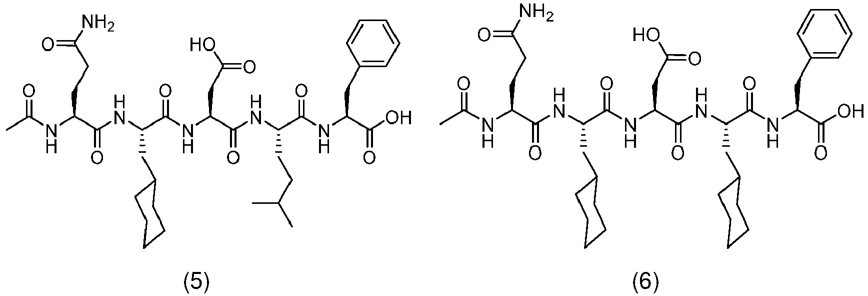

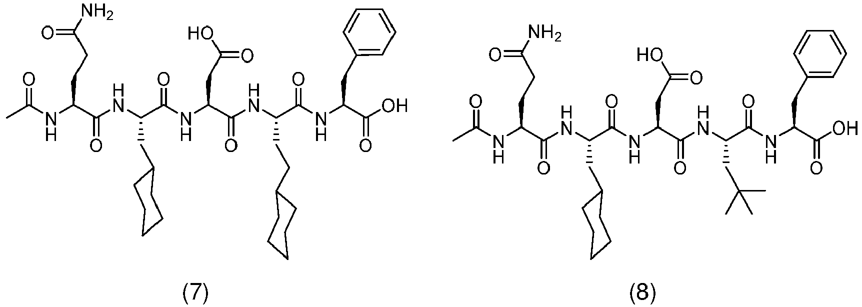

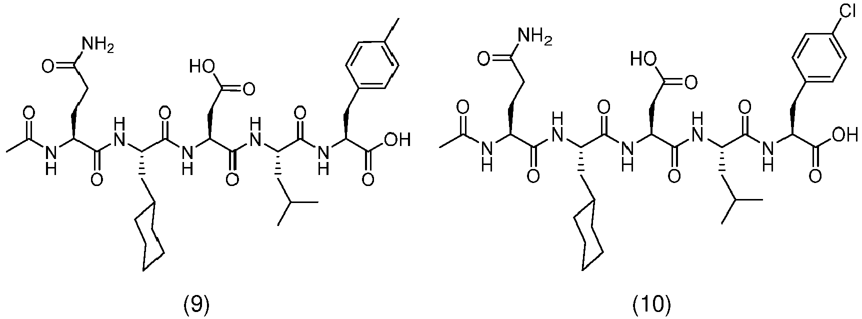

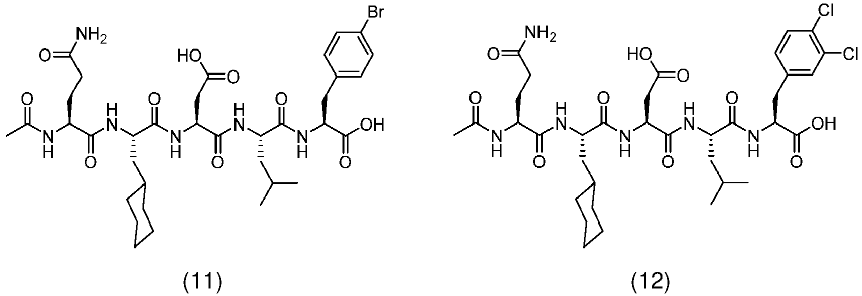

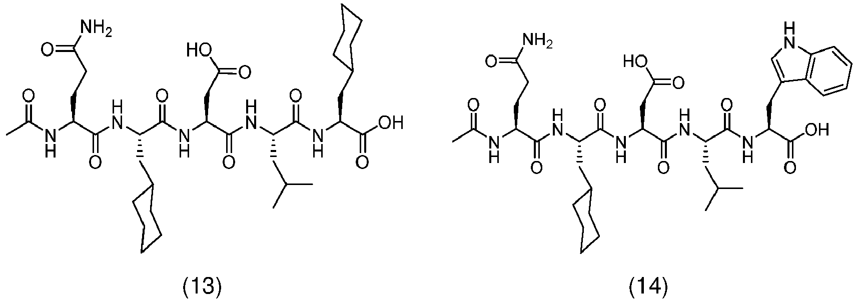

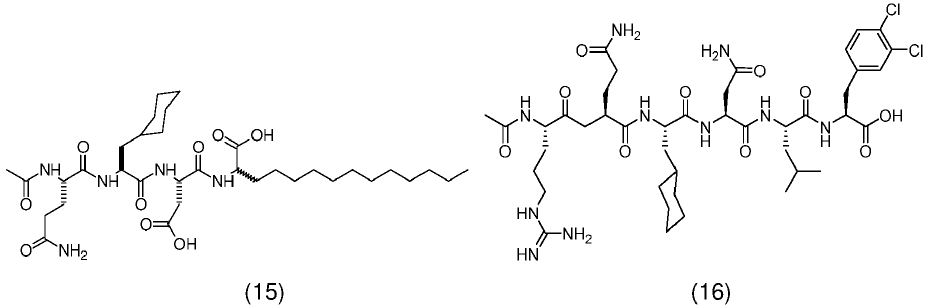

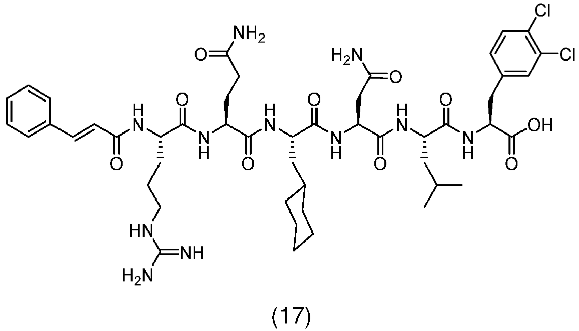

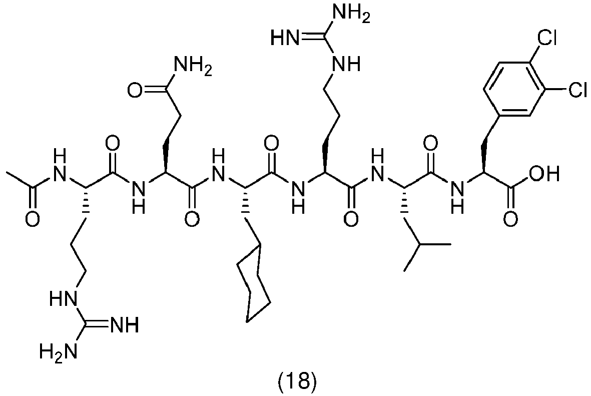

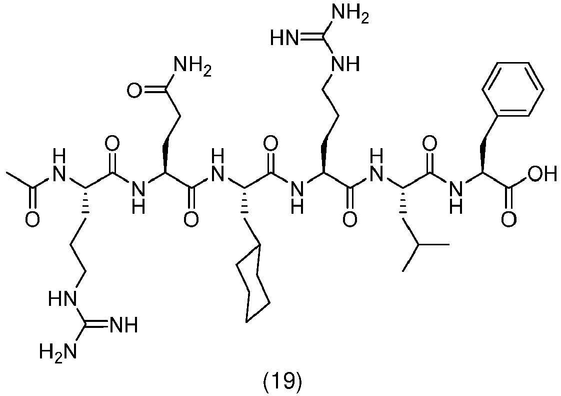

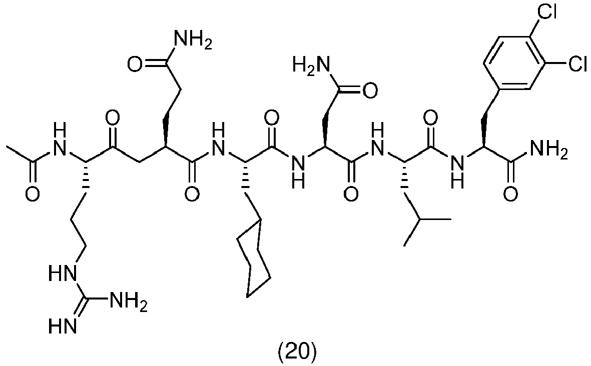

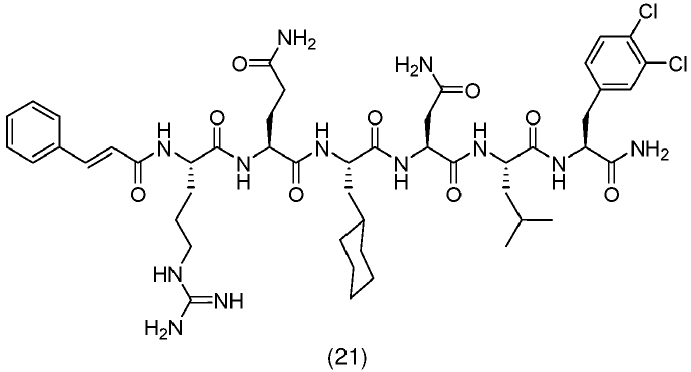

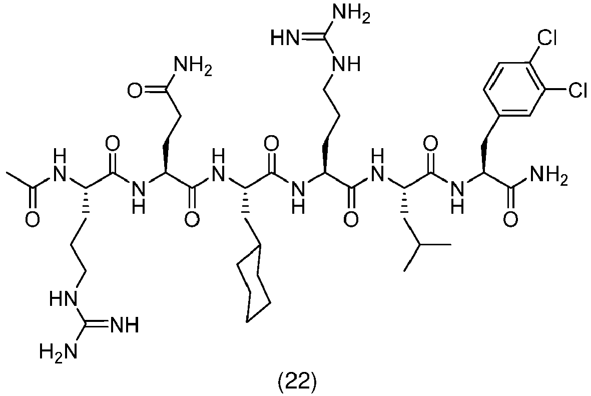

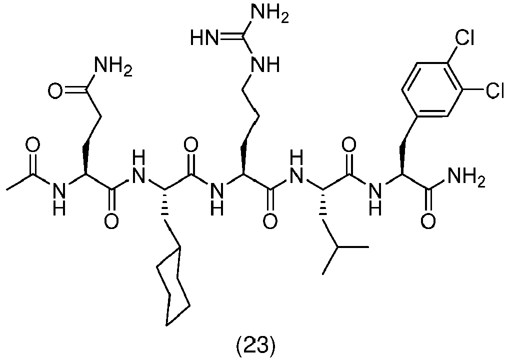

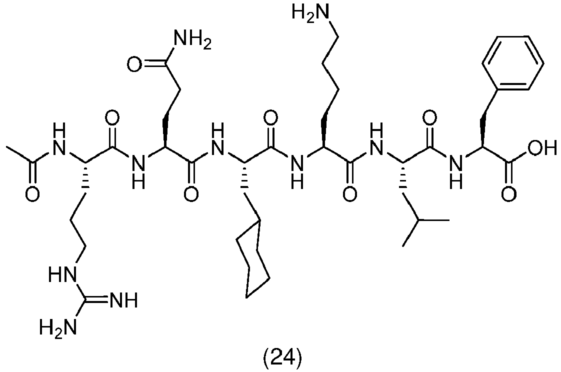

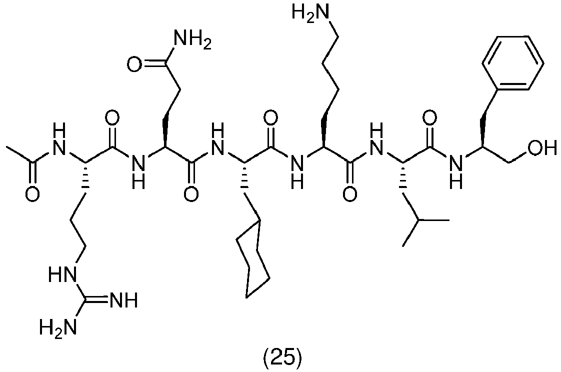

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

The term "heterocycloalkyl" means: a 4- to 10-membered, saturated or partially unsaturated, monocyclic or bicyclic group comprising from one to three heteroatoms selected from O, S or N; the heterocycloalkyl group may be attached to the rest of the molecule via a carbon atom or via a heteroatom; the term bicyclic heterocycloalkyl includes fused bicycles and spiro-type rings.

By way of saturated heterocycloalkyl comprising from 5 to 6 atoms, mention may be made of oxetanyl, tetrahydrofuranyl, dioxolanyl, pyrrolidinyl, azepinyl, oxazepinyl, pyrazolidinyl, imidazolidinyl, tetrahydrothiophenyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

Among the heterocycloalkyls, mention may also be made, by way of examples, of bicyclic groups such as (8aR)-hexahydropyrrolo[1,2-a]pyrazin-2(1 H)-yl, octahydroindozilinyl, diazepanyl, dihydroimidazopyrazinyl and diazabicycloheptanyl groups, or else diazaspiro rings such as 1,7-diazaspiro[4.4]non-7-yl or 1-ethyl-1,7-diazaspiro[4.4]non-7-yl.

When the heterocycloalkyl is substituted, the substitution(s) may be on one (or more) carbon atom(s) and/or on the heteroatom(s). When the heterocycloalkyl comprises several substituents, they may be borne by one and the same atom or different atoms.

The term "(C₂-C₁₂)alkenyl" refers to a branched or straight-chain monovalent unsaturated aliphatic hydrocarbon group having one or more carbon double bonds, of 2 to 12 (inclusive) carbon atoms, preferably 2 to 8 (inclusive) carbon atoms, more preferably 2 to 4 (inclusive) carbon atoms. This term is further exemplified by groups as vinyl, propylenyl, butenyl, pentenyl, hexenyl, heptenyl, octenyl, nonenyl, decenyl, undecenyl, dodecenyl and their straight-chain and branched and stereo isomers. The "alkenyl" group can optionally be mono-, di-, tri- or multiply-substituted by a halogen and/or a C₆₋₁₀-aryl group, as defined below.

The term "cycloalkyl group" means: a cyclic carbon-based group comprising, unless otherwise mentioned, from 3 to 6 carbon atoms. By way of examples, mention may be made of cyclopropyl, cyclobutyl, cyclopentyl, cyclohexyl, etc. groups.

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

The term "halogen" means: a fluorine, a chlorine, a bromine or an iodine.

The term "alkoxy group" means: an -O-alkyl radical where the alkyl group is as previously defined. By way of examples, mention may be made of -O-(C₁-C₄)alkyl groups, and in particular the -O-methyl group, the -O-ethyl group as -O-C₃alkyl group, the -O-propyl group, the -O-isopropyl group, and as -O-C₄alkyl group, the -O-butyl, -O-isobutyl or -O-tert-butyl group.

The abovementioned "alkyl", "cycloalkyl", "aryl", "heteroaryl" and "heterocycloalkyl" radicals can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "carboxyl" means: a COOH group.

The term "oxo" means: "=O".

In some embodiments of the invention, the compounds of the invention can contain one or more asymmetric centers and thus occur as racemates and racemic mixtures, single enantiomers, individual diastereoisomeric mixtures. All such isomeric forms of these compounds are included in the present invention, unless expressly provided otherwise.

In some embodiments, the compounds of the invention can contain one or more double bonds and thus occur as individual or mixtures of Z and/or E isomers. All such isomeric forms of these compounds are included in the present invention, unless expressly provided otherwise.

In the embodiments where the compounds of the invention can contain multiple tautomeric forms, the present invention also includes all tautomeric forms of said compounds unless expressly provided otherwise.

In some embodiments of the invention, the compounds of the invention can be in the form of salts. They can be in non-ionized or in ionized forms.

The present invention is based on different modifications at various positions of two generic peptide sequences. The first group is based on a pentapeptide sequence (AcQ₁X₂D₃L₄F₅, X= Cha) as a primary scaffold and leads to the penta series of peptides (m=0). The second group is based on an hexapeptide sequence (AcQ₁X₂D₃L₄G₅L₆, X= Cha) and derives from the natural SC binding peptide sequence of *E.coli* DNA polymerase IV.

According to an embodiment, in formula (I), m=0. This specific family of compounds is made of pentapeptides.

According to an embodiment, in formula (I), m=1. This specific family of compounds is made of hexapeptides.

A preferred group of compounds according to the invention are compounds of formula (I) as defined above wherein m=0 and at least one of Rₐ, R_{b}, R_{c}, R_{d}, Rₑ, and R_{f} is not H. Such preferred group of compounds comprises pentapeptides wherein at least one residue comprises at least one Rₐ, R_{b}, R_{c}, R_{d}, Rₑ, or R_{f} group being -CH₂-CH=CH₂, a (C₁-C₆)alkyl group, a (C₆-C₁₀)aryl group or an aralkyl group.

Another preferred group of is defined by compounds of formula (I) wherein m=0 and at least one Rₐ, R_{b}, R_{c}, R_{d}, Rₑ, or R_{f} group is -CH₂-CH=CH₂ or a (C₁-C₆)alkyl group, preferably a methyl group.

Another preferred group of is defined by compounds of formula (I) wherein m=0 and Rₐ, R_{b}, R_{d}, Rₑ, and R_{f} are H and R_{c} is Me.

According to an embodiment, in formula (I), Rₐ, R_{b}, R_{d}, Rₑ, and R_{f} are H and R_{c} is Me.

The present invention also relates to compounds having the following formula (III-1): 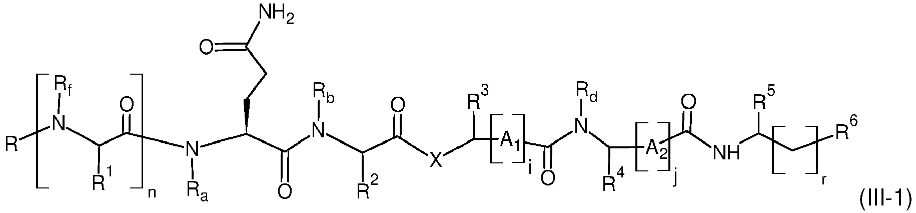 wherein:
- n is 0 or 1;
- i is 0 or 1;
- j is 0 or 1;
- r is 0, 1 or 2;
- R is selected in the group consisting of:
   * a (C₁-C₁₂)alkyl group optionally substituted by a (C₆-C₁₀)aryl group, by a heteroaryl group or by a heterocycloalkyl group,
   * a (C₂-Ci₂)alkenyl group optionally substituted by a C₆₋₁₀-aryl group,
   * a (C₃-C₆)cycloalkyl group,
   * a (C₆-C₁₀)aryl group optionally substituted by a C₁₋₄-alkyl,
   * a group having the following formula (II): 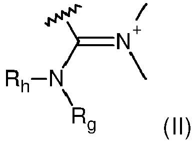 R_{g} and Rₕ representing independently from each other a (C₁-C₆)alkyl group or forming together with the nitrogen atom carrying them a heterocycloalkyl group,
   * a group -C(=O)-R', R' being selected in the group consisting of:
      - a (C₁-C₁₂)alkyl group optionally substituted by an optionally substituted (C₆-C₁₀)aryl group,
      - a (C₂-Ci₂)alkenyl group optionally substituted by an optionally substituted C₆₋₁₀-aryl group or by a heteroaryl group,
      - a -NH-optionally substituted (C₆-C₁₀)aryl group,
      - a (C₃-C₆)cycloalkyl group,
      - a (C₆-C₁₀)aryl group optionally substituted by a C₁₋₄-alkyl,
   * a group having the following formula (II-1): 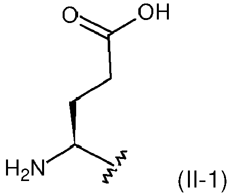
- X is O or a group N-R_{c};
- Rₐ, R_{b}, R_{c}, R_{d}, and R_{f} are, independently from each other, selected from the group consisting of: H, -CH₂-CH=CH₂, (C₁-C₆)alkyl groups, (C₆-C₁₀)aryl groups, and aralkyl groups;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
   or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
   * q is 1, 2, 3 or 4,
   * R^{7a} is a hydrogen atom, a (C₁-C₈)alkyl group, or an aralkyl group;
   * R^{7b} is a hydrogen atom, a (C₁-C₈)alkyl group, or an aralkyl group,
   * R⁸ is a hydrogen atom, a (C₁-C₈)alkyl group, or an aralkyl group, and
   * R⁹ is H or COR¹¹, R¹¹ being H or a (C₁-C₈)alkyl group, such as CH₃;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, such as being Me, and R'¹² being selected from the group consisting of: H, (C₁-C₈)alkyl group such as Me, and -C(=O)-(C₁-C₈)alkyl group, such as -C(=O)-CH₃;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)_{S}-, s being 1, 2, 3 or 4;
- R⁵ is selected in the group consisting of:
   * a -(CH₂)-(C₃-C₆)cycloalkyl group;
   * a -(CH₂-CH₂)-(C₃-C₆)cycloalkyl group;
   * a -(CH₂)-(C₆-C₁₀)aryl group optionally substituted by at least one halogen, (C₁-C₂)alkyl group and/or (C₁-C₂)alkoxy group;
   * a -(CH₂-CH₂)-(C₆-C₁₀)aryl group optionally substituted by at least one halogen, (C₁-C₂)alkyl group and/or (C₁-C₂)alkoxy group;
   * a -(CH₂)-(C₅-C₁₀)heteroaryl group optionally substituted by at least one halogen and/or (C₁-C₂)alkyl group; and
   * a -(CH₂-CH₂)-(C₅-C₁₀)heteroaryl group optionally substituted by at least one halogen and/or (C₁-C₂)alkyl group;
   * a (C₁-C₈)alkyl group or a (C₁-C₄)alkyl group optionally substituted by at least one halogen;
- R⁶ is H, -CO₂H, -CO₂R₁₀, -CO-NH₂, -CO-NHR₁₀, -OR¹⁰, -OH or NH₂ when r is 0, 1 or 2, -NH-CO-NHR¹⁰ when r is 1 or 2; wherein
   * R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group,
the compounds (1), (2), (3), (4), (5), (6), (7), (8), (9), (10), (11), (12), (13), (14), (15), (16), (17), (18), (19), (20), (21), (22), (23), (24), and (25) as defined above being excluded.

The present invention also relates to compounds having the following formula (III) : 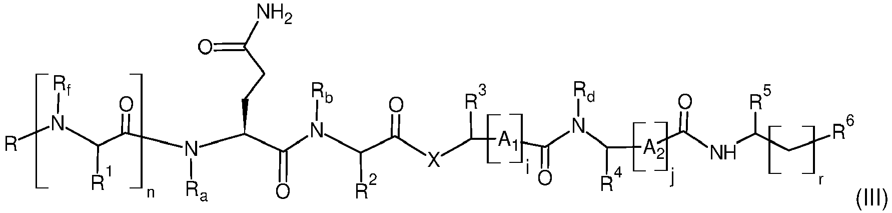 wherein:
- n is 0 or 1;
- i is 0 or 1;
-j is 0 or 1;
- r is 0, 1 or 2;
- R is selected in the group consisting of:
   * a (C₁-C₁₂)alkyl group optionally substituted by a (C₆-C₁₀)aryl group, by a heteroaryl group or by a heterocycloalkyl group,
   * a (C₂-Ci₂)alkenyl group optionally substituted by a C₆₋₁₀-aryl group,
   * a (C₃-C₆)cycloalkyl group,
   * a (C₆-C₁₀)aryl group optionally substituted by a C₁₋₄-alkyl,
   * a group having the following formula (II): 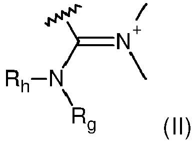 R_{g} and Rₕ representing independently from each other a (C₁-C₆)alkyl group or forming together with the nitrogen atom carrying them a heterocycloalkyl group,
   * a group -C(=O)-R', R' being selected in the group consisting of:
      - a (C₁-C₁₂)alkyl group optionally substituted by an optionally substituted (C₆-C₁₀)aryl group,
      - a (C₂-C₁₂)alkenyl group optionally substituted by an optionally substituted C₆₋₁₀-aryl group or by a heteroaryl group,
      - a -NH-optionally substituted (C₆-C₁₀)aryl group,
      - a (C₃-C₆)cycloalkyl group,
      - a (C₆-C₁₀)aryl group optionally substituted by a C₁₋₄-alkyl,
   * a group having the following formula (II-1): 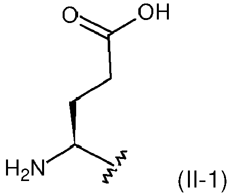
- X is O or a group N-R_{c};
- Rₐ, R_{b}, R_{c}, R_{d}, and R_{f} are, independently from each other, selected from the group consisting of: H, -CH₂-CH=CH₂, (C₁-C₆)alkyl groups, (C₆-C₁₀)aryl groups, and aralkyl groups, at least one of Rₐ, R_{b}, R_{c}, R_{d}, and R_{f} being not H;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
   or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
   * q is 1, 2, 3 or 4,
   * R^{7a} is a hydrogen atom, a (C₁-C₈)alkyl group, or an aralkyl group;
   * R^{7b} is a hydrogen atom, a (C₁-C₈)alkyl group, or an aralkyl group,
   * R⁸ is a hydrogen atom, a (C₁-C₈)alkyl group, or an aralkyl group, and
   * R⁹ is H or COR¹¹, R¹¹ being H or a (C₁-C₈)alkyl group, such as CH₃;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, such as being Me, and R'¹² being selected from the group consisting of: H, (C₁-C₈)alkyl group such as Me, and -C(=O)-(C₁-C₈)alkyl group, such as -C(=O)-CH₃;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)_{S}-, s being 1, 2, 3 or 4;
- R⁵ is selected in the group consisting of:
   * a -(CH₂)-(C₃-C₆)cycloalkyl group;
   * a -(CH₂-CH₂)-(C₃-C₆)cycloalkyl group;
   * a -(CH₂)-(C₆-C₁₀)aryl group optionally substituted by at least one halogen, (C₁-C₂)alkyl group and/or (C₁-C₂)alkoxy group;
   * a -(CH₂-CH₂)-(C₆-C₁₀)aryl group optionally substituted by at least one halogen, (C₁-C₂)alkyl group and/or (C₁-C₂)alkoxy group;
   * a -(CH₂)-(C₅-C₁₀)heteroaryl group optionally substituted by at least one halogen and/or (C₁-C₂)alkyl group; and
   * a -(CH₂-CH₂)-(C₅-C₁₀)heteroaryl group optionally substituted by at least one halogen and/or (C₁-C₂)alkyl group;
   * a (C₁-C₈)alkyl group or a (C₁-C₄)alkyl group optionally substituted by at least one halogen;
- R⁶ is H, -CO₂H, -CO₂R₁₀, -CO-NH₂, -CO-NHR₁₀, -OR¹⁰, -OH or NH₂ when r is 0, 1 or 2, -NH-CO-NHR¹⁰ when r is 1 or 2; wherein
   * R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group.

The compounds having the above formula (III) and (III-1) are compounds of the pentapeptides group.

The present invention also relates to compounds of formula (IV): 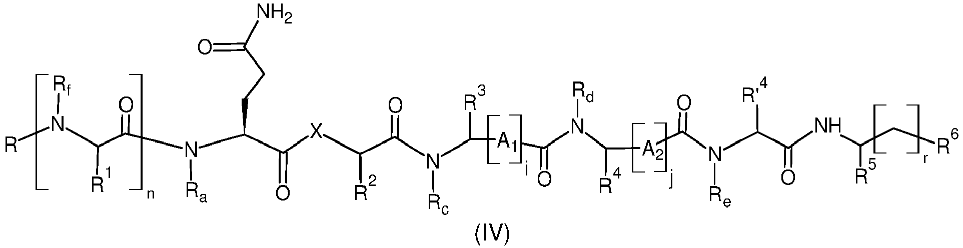 wherein:
- n is 0 or 1;
- i is 0 or 1;
- j is 0 or 1;
- r is 0, 1 or 2;
- R is selected in the group consisting of:
   * a (C₁-C₁₂)alkyl group optionally substituted by a (C₆-C₁₀)aryl group, by a heteroaryl group or by a heterocycloalkyl group,
   * a (C₂-Ci₂)alkenyl group optionally substituted by a (C₆-C₁₀)aryl group,
   * a (C₃-C₆)cycloalkyl group,
   * a (C₆-C₁₀)aryl group optionally substituted by a (C₁-C₄)alkyl,
   * a group having the following formula (II): 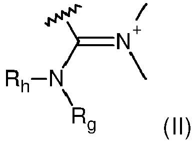 R_{g} and Rₕ representing independently from each other a (C₁-C₆)alkyl group or forming together with the nitrogen atom carrying them a heterocycloalkyl group,
   * a group -C(=O)-R', R' being selected in the group consisting of:
      - a (C₁-C₁₂)alkyl group optionally substituted by an optionally substituted (C₆-C₁₀)aryl group,
      - a (C₂-Ci₂)alkenyl group optionally substituted by an optionally substituted C₆₋₁₀-aryl group or by a heteroaryl group,
      - a -NH-optionally substituted (C₆-C₁₀)aryl group,
      - a (C₃-C₆)cycloalkyl group,
      - a (C₆-C₁₀)aryl group optionally substituted by a (C₁-C₄)alkyl,
   * a group having the following formula (II-1): 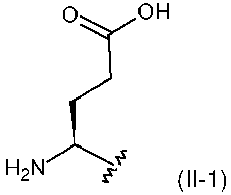
- X is O or a group N-R_{c};
- Rₐ, R_{b}, R_{c}, R_{d}, Rₑ, and R_{f} are, independently from each other, selected from the group consisting of: H, -CH₂-CH=CH₂, (C₁-C₆)alkyl groups, (C₆-C₁₀)aryl groups, and aralkyl groups;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
   or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
   * q is 1, 2, 3 or 4,
   * R^{7a} is a hydrogen atom, a (C₁-C₈)alkyl group, or an aralkyl group;
   * R^{7b} is a hydrogen atom, a (C₁-C₈)alkyl group, or an aralkyl group,
   * R⁸ is a hydrogen atom, a (C₁-C₈)alkyl group, or an aralkyl group, and
   * R⁹ is H or COR¹¹, R¹¹ being H or a (C₁-C₈)alkyl group, such as CH₃;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, such as being Me, and R'¹² being selected from the group consisting of: H, (C₁-C₈)alkyl group such as Me, and -C(=O)-(C₁-C₈)alkyl group, such as -C(=O)-CH₃;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)_{S}-, s being 1, 2, 3 or 4;
- R'⁴ is H, a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
   or R'⁴ forms a heterocycloalkyl group with Rₑ, the carbon atom carrying it and the nitrogen atom carrying Rₑ, said heterocycloalkyl group being optionally substituted with at least one hydroxyl group, amino group, or sulfur atom;
- R⁵ is selected in the group consisting of:
   * a -(CH₂)-(C₃-C₆)cycloalkyl group;
   * a -(CH₂-CH₂)-(C₃-C₆)cycloalkyl group;
   * a -(CH₂)-(C₆-C₁₀)aryl group optionally substituted by at least one halogen, (C₁-C₂)alkyl group and/or (C₁-C₂)alkoxy group;
   * a -(CH₂-CH₂)-(C₆-C₁₀)aryl group optionally substituted by at least one halogen, (C₁-C₂)alkyl group and/or (C₁-C₂)alkoxy group;
   * a -(CH₂)-(C₅-C₁₀)heteroaryl group optionally substituted by at least one halogen and/or (C₁-C₂)alkyl group;
   * a -(CH₂-CH₂)-(C₅-C₁₀)heteroaryl group optionally substituted by at least one halogen and/or (C₁-C₂)alkyl group; and
   * a (C₁-C₈)alkyl group or a (C₁-C₄)alkyl group optionally substituted by at least one halogen;
- R⁶ is H, -CO₂H, -CO₂R₁₀, -CO-NH₂, -CO-NHR₁₀, -OR¹⁰, -OH or NH₂ when r is 0 or 1 or 2, -NH-CO-NHR¹⁰ when r is 1 or 2; wherein
   * R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group.

The compounds having the above formula (IV) are compounds of the hexapeptides group.

A preferred group of compounds according to the invention comprises compounds of formula (III), (III-1) or (IV) wherein R is selected in the group consisting of:
* a (C₁-C₁₂)alkyl group optionally substituted by a (C₆-C₁₀)aryl group, by a heteroaryl group or by a heterocycloalkyl group,
* a group having the following formula (II): 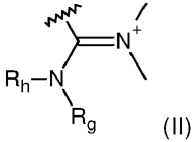 R_{g} and Rₕ representing independently from each other a (C₁-C₆)alkyl group or forming together with the nitrogen atom carrying them a heterocycloalkyl group,
* a group -C(=O)-R', R' being selected in the group consisting of:
   - a (C₁-C₁₂)alkyl group optionally substituted by an optionally substituted (C₆-C₁₀)aryl group,
   - a (C₂-Ci₂)alkenyl group optionally substituted by an optionally substituted C₆₋₁₀-aryl group or by a heteroaryl group,
   - a -NH-optionally substituted (C₆-C₁₀)aryl group, and
* a group having the following formula (II-1):

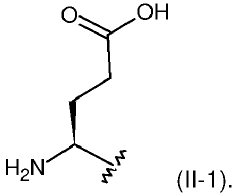

According to a preferred embodiment, in formula (III), (III-1) or (IV), R is a group -C(=O)-R', R' being selected in the group consisting of:
- a (C₁-C₁₂)alkyl group optionally substituted by an optionally substituted (C₆-C₁₀)aryl group,
- a (C₂-Ci₂)alkenyl group optionally substituted by an optionally substituted C₆₋₁₀-aryl group or by a heteroaryl group, and
- a -NH-optionally substituted (C₆-C₁₀)aryl group.

According to a preferred embodiment, in formula (III), (III-1) or (IV), R is a group -C(=O)-R', R' being:
- either a (C₁-C₁₂)alkyl group optionally substituted by an optionally substituted (C₆-C₁₀)aryl group,
- or a (C₂-Ci₂)alkenyl group optionally substituted by an optionally substituted C₆₋₁₀-aryl group or by a heteroaryl group.

According to a preferred embodiment, in formula (III), (III-1) or (IV), R is a group -C(=O)-R', R' being a (C₁-C₁₂)alkyl group.

A preferred group of compounds according to the invention comprises compounds of formula (III) wherein R is a group -C(=O)-R', R' being a (C₁-C₁₂)alkyl group, preferably a methyl group.

A preferred group of compounds according to the invention comprises compounds of formula (III), (III-1) or (IV) wherein R² is a -CH₂-(C₃-C₆)cycloalkyl group, preferably a -CH₂-cyclohexyl group.

A preferred group of compounds according to the invention comprises compounds of formula (III), (III-1) or (IV), wherein R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

A preferred group of compounds according to the invention comprises compounds of formula (III), (III-1) or (IV), wherein R⁴ is a (C₁-C₈)alkyl group.

A preferred group of compounds according to the invention comprises compounds of formula (III), (III-1) or (IV), wherein R⁵ is a -(CH₂)-(C₆-C₁₀)aryl group optionally substituted by at least one halogen, (C₁-C₂)alkyl group and/or (C₁-C₂)alkoxy group, or a (C₁-C₈)alkyl group or a (C₁-C₄)alkyl group optionally substituted by at least one halogen.

A preferred group of compounds according to the invention comprises compounds of formula (III) or (III-1) wherein R⁵ is a -(CH₂)-(C₆-C₁₀)aryl group optionally substituted by at least one halogen.

A preferred group of compounds according to the invention comprises compounds of formula (III) or (III-1) wherein R⁵ is a -(CH₂)-(C₆-C₁₀)aryl group, optionally substituted by at least one halogen, R⁵ being in particular a benzyl group, optionally substituted by at least one halogen, preferably by at least two halogen atoms, such as chlorine.

A preferred group of compounds according to the invention comprises compounds of formula (IV), wherein R⁵ is a (C₁-C₈)alkyl group or a -(CH₂)-(C₆-C₁₀)aryl group optionally substituted by at least one halogen.

A preferred group of compounds according to the invention comprises compounds of formula (III), (III-1) or (IV), wherein R⁶ is H, -CO₂H, -CO-NH₂, or - CO₂R¹⁰, R¹⁰ being a (C₁-C₈)alkyl group, preferably Me, or R⁶ is -OH or NH₂ when r is 1.

A preferred group of compounds according to the invention comprises compounds of formula (III) or (III-1), wherein R⁶ is COOH.

A preferred group of compounds according to the invention comprises compounds of formula (IV), wherein R⁶ is COOH.

A preferred group of compounds according to the invention comprises compounds of formula (III), (III-1) or (IV), wherein R'⁴ is H or forms a heterocycloalkyl group with Rₑ, the carbon atom carrying it and the nitrogen atom carrying Rₑ, such as for example a pyrrolidine or a piperidine or octahydroindole ring, said heterocycloalkyl group being optionally substituted with at least one hydroxyl group.

According to an embodiment, in formula (III), (III-1) or (IV), R'⁴ forms a octahydroindole group (Oic) with Rₑ, the carbon atom carrying it and the nitrogen atom carrying Rₑ.

The present invention also relates to compounds having the following formula (V):

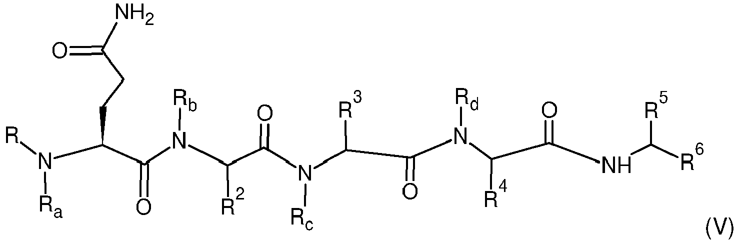

R, Rₐ, R_{b}, R_{c}, R_{d}, R², R³, R⁴, R⁵, and R⁶ being as defined above in formula (I) or (III).

Preferably, in formula (V), R is a group -C(=O)-R', R' being selected in the group consisting of:
- a (C₁-C₁₂)alkyl group optionally substituted by an optionally substituted (C₆-C₁₀)aryl group,
- a (C₂-Ci₂)alkenyl group optionally substituted by an optionally substituted C₆₋₁₀-aryl group or by a heteroaryl group, and
- a -NH-optionally substituted (C₆-C₁₀)aryl group.

According to a preferred embodiment, in formula (V), R is a group -C(=O)-R', R' being a (C₁-C₁₂)alkyl group, preferably a methyl group.

Preferably, in formula (V), R² is a -CH₂-(C₃-C₆)cycloalkyl group, preferably a -CH₂-cyclohexyl group.

Preferably, in formula (V), R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

Preferably, in formula (V), R³ is -CH₂-COOH.

Preferably, in formula (V), R⁴ is a (C₁-C₈)alkyl group.

Preferably, in formula (V), R⁵ is a -(CH₂)-(C₆-C₁₀)aryl group optionally substituted by at least one halogen.

Preferably, in formula (V), R⁶ is COOH.

A preferred group of compounds according to the invention comprises compounds of formula (V) wherein:
- R is a group -C(=O)-R', R' being selected in the group consisting of:
   - a (C₁-C₁₂)alkyl group optionally substituted by an optionally substituted (C₆-C₁₀)aryl group,
   - a (C₂-Ci₂)alkenyl group optionally substituted by an optionally substituted C₆₋₁₀-aryl group or by a heteroaryl group, and
   - a -NH-optionally substituted (C₆-C₁₀)aryl group;
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁴ is a (C₁-C₈)alkyl group;
- R⁵ is a -(CH₂)-(C₆-C₁₀)aryl group optionally substituted by at least one halogen; and
- R⁶ is COOH.

The present invention also relates to compounds having the following formula (VI):

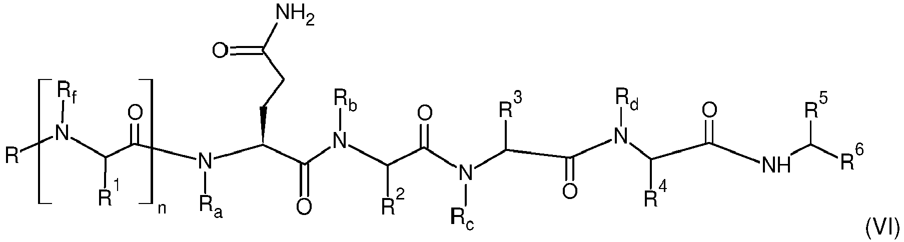

n, R, Rₐ, R_{b}, R_{c}, R_{d}, R_{f}, R¹, R², R³, R⁴, R⁵, and R⁶ being as defined above in formula (I) or (III).

Preferably, in formula (VI), R is different from -C(=O)Me.

Preferably, in formula (VI), R is different from a transcinnamoyl group when R is a dichlorophenyl group (3,4-diCIPhe).

Preferably, in formula (VI), n=1 and R¹ is H. Preferably, in formula (VI), n=1, R¹ is H and R_{f} is H. More preferably, in formula (VI), R is a group of formula (II) as defined above, wherein in particular R_{g} and Rₕ are alkyl groups, and more preferably R_{g}=Rₕ=Me.

According to a preferred embodiment, in formula (VI), n=1, R¹ is H, R_{f} is H, and R is a group of formula (II) as defined above, wherein in particular R_{g} and Rₕ are alkyl groups, and more preferably R_{g}=Rₕ=Me.

Preferably, in formula (VI), R² is a -CH₂-(C₃-C₆)cycloalkyl group, preferably a -CH₂-cyclohexyl group.

Preferably, in formula (VI), R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R³ is a hydrogen atom or a (C₁-C₈)alkyl group.

Preferably, in formula (VI), R³ is -CH₂-COOH.

Preferably, in formula (VI), R⁴ is a (C₁-C₈)alkyl group.

Preferably, in formula (VI), R⁵ is a -(CH₂)-(C₆-C₁₀)aryl group optionally substituted by at least one halogen.

Preferably, in formula (VI), R⁶ is COOH.

A preferred group of compounds according to the invention comprises compounds of formula (VI) wherein:
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁴ is a (C₁-C₈)alkyl group;
- R⁵ is a -(CH₂)-(C₆-C₁₀)aryl group optionally substituted by at least one halogen; and
- R⁶ is COOH.

A preferred group of compounds according to the invention comprises compounds of formula (VI) wherein:
- R is not a -C(=O)Me group;
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁴ is a (C₁-C₈)alkyl group;
- R⁵ is a -(CH₂)-(C₆-C₁₀)aryl group optionally substituted by at least one halogen; and
- R⁶ is COOH.

The present invention also relates to compounds having the following formula (VII):

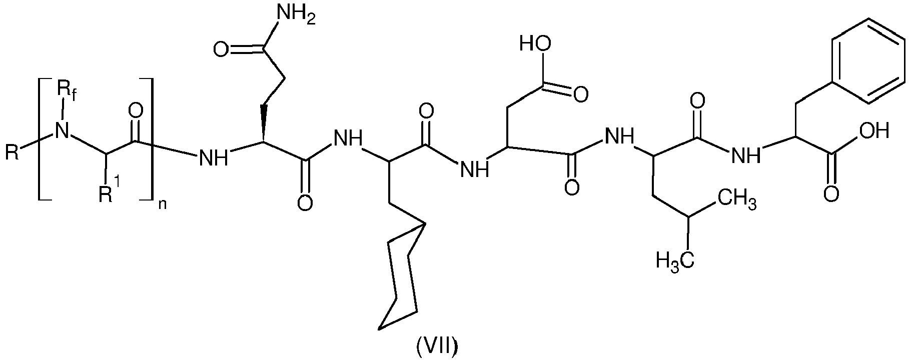

n, R, R_{f}, and R¹ being as defined above in formula (I) or (III).

Preferably, in formula (VII), R_{f} is H.

Preferably, in formula (VII), R is different from -C(=O)Me.

The present invention also relates to compounds having the following formula (VIII):

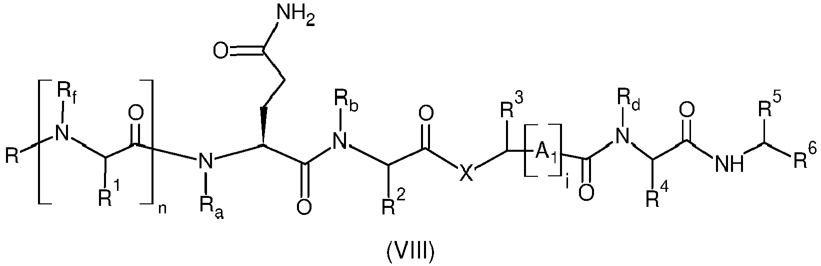

n, R, Rₐ, R_{b}, R_{d}, R_{f}, i, X, A₁, R¹, R², R³, R⁴, R⁵, and R⁶ being as defined above in formula (I) or (III).

The present invention also relates to compounds having the following formula (IX):

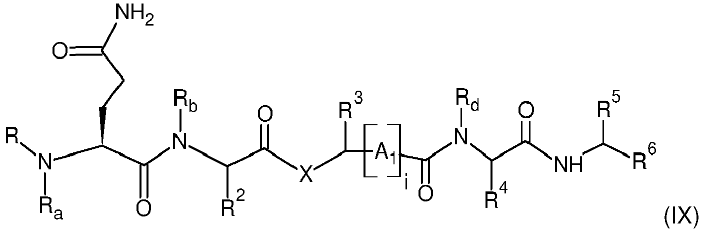

R, Rₐ, R_{b}, R_{d}, i, X, A₁, R², R³, R⁴, R⁵, and R⁶ being as defined above in formula (I) or (III).

Preferably, in formula (VIII) or (IX), R² is a -CH₂-(C₃-C₆)cycloalkyl group, preferably a -CH₂-cyclohexyl group.

Preferably, in formula (VIII) or (IX), R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

Preferably, in formula (VIII) or (IX), R³ is different from -CH₂COOH.

Preferably, in formula (VIII) or (IX), R⁴ is a (C₁-C₈)alkyl group.

Preferably, in formula (VIII) or (IX), R⁵ is a -(CH₂)-(C₆-C₁₀)aryl group optionally substituted by at least one halogen.

Preferably, in formula (VIII) or (IX), R⁶ is COOH or CONH₂.

Preferably, in formula (IX), R is a group -C(=O)-R', R' being selected in the group consisting of:
- a (C₁-C₁₂)alkyl group optionally substituted by an optionally substituted (C₆-C₁₀)aryl group,
- a (C₂-Ci₂)alkenyl group optionally substituted by an optionally substituted C₆₋₁₀-aryl group or by a heteroaryl group, and
- a -NH-optionally substituted (C₆-C₁₀)aryl group.

According to a preferred embodiment, in formula (IX), R is a group -C(=O)-R', R' being a (C₁-C₁₂)alkyl group, preferably a methyl group.

A preferred group of compounds according to the invention comprises compounds of formula (VIII) or (IX), wherein:
- R² is a -CH₂-(C₃-C₆)cycloalkyl group, preferably a -CH₂-cyclohexyl group;
- R⁴ is a (C₁-C₈)alkyl group;
- R⁵ is a -(CH₂)-(C₆-C₁₀)aryl group optionally substituted by at least one halogen; and
- R⁶ is COOH.

A preferred group of compounds according to the invention comprises compounds of formula (IX), wherein:
- R is a group -C(=O)-R', R' being a (C₁-C₁₂)alkyl group, preferably a methyl group;
- R² is a -CH₂-(C₃-C₆)cycloalkyl group, preferably a -CH₂-cyclohexyl group;
- R⁴ is a (C₁-C₈)alkyl group;
- R⁵ is a -(CH₂)-(C₆-C₁₀)aryl group optionally substituted by at least one halogen; and
- R⁶ is COOH.

Preferably, in formula (VIII) or (IX), Rₐ=R_{b}=R_{d}=H.

The present invention also relates to compounds having the following formula (X):

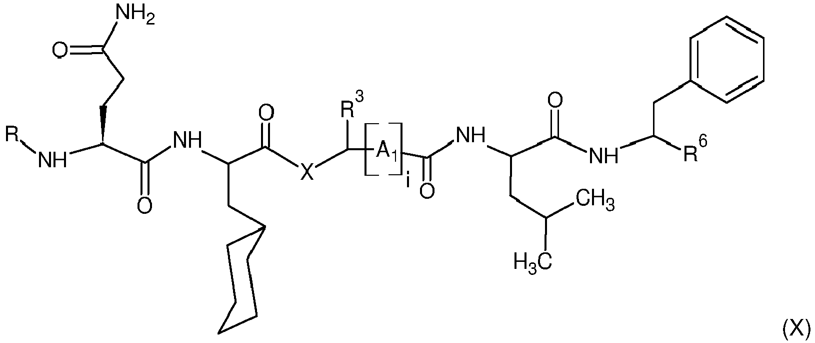

R, i, X, A₁, R³, and R⁶ being as defined above in formula (I) or (III).

Preferably, in formula (X), R⁶ is COOH, COOEt, or CONH₂.

Preferably, in formula (X), R is -COMe.

Preferably, in formula (X), when X is NH and i=0, R³ is different from - CH₂COOH.

The present invention also relates to compounds having the following formula (XI):

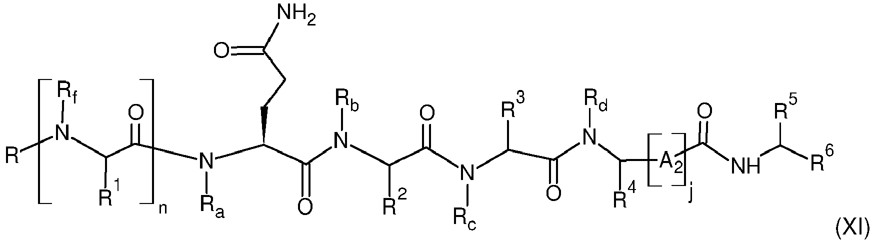

n, R, Rₐ, R_{b}, R_{c}, R_{d}, R_{f}, j, A₂, R¹, R², R³, R⁴, R⁵, and R⁶ being as defined above in formula (I) or (III).

The present invention also relates to compounds having the following formula (XII):

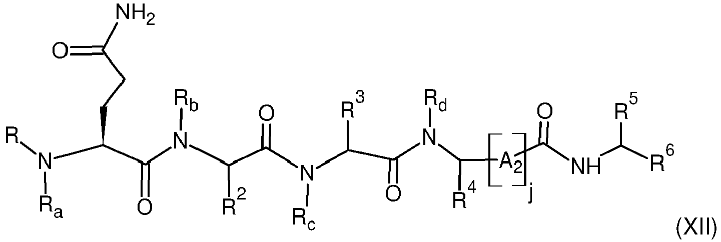

R, Rₐ, R_{b}, R_{c}, R_{d}, j, A₂, R², R³, R⁴, R⁵, and R⁶ being as defined above in formula (I) or (III).

Preferably, in formula (XI) or (XII), R² is a -CH₂-(C₃-C₆)cycloalkyl group, preferably a -CH₂-cyclohexyl group.

Preferably, in formula (XI) or (XII), R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

More preferably, in formula (XI) or (XII), R³ is -CH₂-COOH.

Preferably, in formula (XI) or (XII), R⁴ is a (C₁-C₈)alkyl group, more preferably different from isopropyl when j=0.

According to an embodiment, in formula (XI) or (XII), j=1 and A₂ is a -CH₂-CH₂-radical.

Preferably, in formula (XI) or (XII), R⁵ is a -(CH₂)-(C₆-C₁₀)aryl group optionally substituted by at least one halogen.

Preferably, in formula (XI) or (XII), R⁶ is COOH.

Preferably, in formula (XII), R is a group -C(=O)-R', R' being selected in the group consisting of:
- a (C₁-C₁₂)alkyl group optionally substituted by an optionally substituted (C₆-C₁₀)aryl group,
- a (C₂-Ci₂)alkenyl group optionally substituted by an optionally substituted C₆₋₁₀-aryl group or by a heteroaryl group, and
- a -NH-optionally substituted (C₆-C₁₀)aryl group.

According to a preferred embodiment, in formula (XII), R is a group -C(=O)-R', R' being a (C₁-C₁₂)alkyl group, preferably a methyl group.

A preferred group of compounds according to the invention comprises compounds of formula (XI) or (XII), wherein:
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁵ is a -(CH₂)-(C₆-C₁₀)aryl group optionally substituted by at least one halogen; and
- R⁶ is COOH.

A preferred group of compounds according to the invention comprises compounds of formula or (XII), wherein:
- R is a group -C(=O)-R', R' being a (C₁-C₁₂)alkyl group, preferably a methyl group;
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁵ is a -(CH₂)-(C₆-C₁₀)aryl group optionally substituted by at least one halogen; and
- R⁶ is COOH.

Preferably, in formula (XI) or (XII), Rₐ=R_{b}=R_{c}=R_{d}=H.

The present invention also relates to compounds having the following formula (XIII):

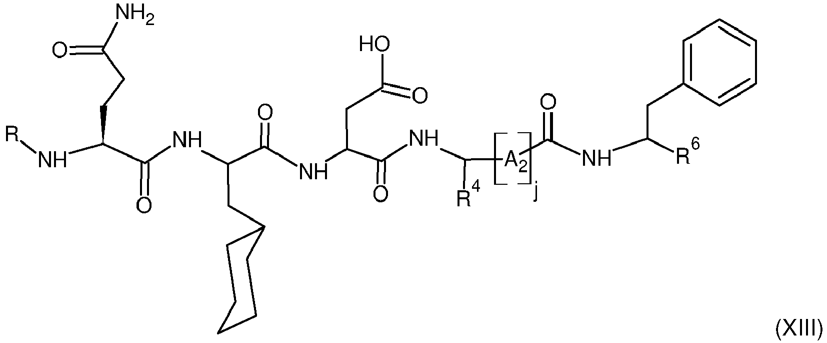

R, j, A₂, R⁴, and R⁶ being as defined above in formula (I) or (III).

Preferably, in formula (XIII), R⁶ is COOH.

Preferably, in formula (XIII), R is -COMe.

Preferably, in formula (XIII), when j=0, R⁴ is different from isopropyl.

The present invention also relates to compounds having the following formula (XIV):

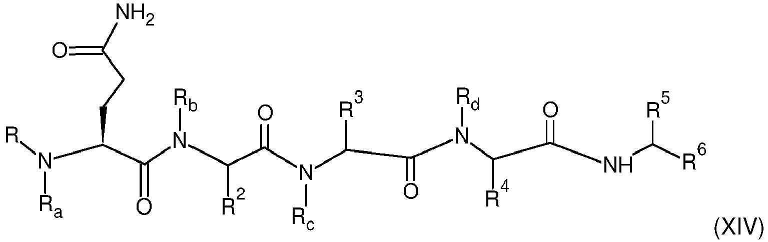

R, Rₐ, R_{b}, R_{c}, R_{d}, R², R³, R⁴, R⁵, and R⁶ being as defined above in formula (I) or (III).

Preferably, in formula (XIV), R is a group -C(=O)-R', R' being selected in the group consisting of:
- a (C₁-C₁₂)alkyl group optionally substituted by an optionally substituted (C₆-C₁₀)aryl group,
- a (C₂-C₁₂)alkenyl group optionally substituted by an optionally substituted C₆₋₁₀-aryl group or by a heteroaryl group, and
- a -NH-optionally substituted (C₆-C₁₀)aryl group.

According to a preferred embodiment, in formula (XIV), R is a group -C(=O)-R', R' being a (C₁-C₁₂)alkyl group, preferably a methyl group.

Preferably, in formula (XIV), R² is a -CH₂-(C₃-C₆)cycloalkyl group, preferably a -CH₂-cyclohexyl group.

Preferably, in formula (XIV), R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

Preferably, in formula (XIV), R⁴ is a (C₁-C₈)alkyl group.

Preferably, in formula (XIV), Rₐ=R_{b}=R_{c}=R_{d}=H.

A preferred group of compounds according to the invention comprises compounds of formula (XIV), wherein R⁵ is a -(CH₂)-(C₆-C₁₀)aryl group optionally substituted by at least one halogen, (C₁-C₂)alkyl group and/or (C₁-C₂)alkoxy group, or a (C₁-C₈)alkyl group or a (C₁-C₄)alkyl group optionally substituted by at least one halogen, or a -(CH₂-CH₂)-(C₆-C₁₀)aryl group optionally substituted by at least one halogen, (C₁-C₂)alkyl group and/or (C₁-C₂)alkoxy group.

A preferred group of compounds according to the invention comprises compounds of formula (XIV), wherein R⁵ is a -(CH₂)-(C₆-C₁₀)aryl group optionally substituted by at least one halogen, (C₁-C₂)alkyl group and/or (C₁-C₂)alkoxy group, or a -(CH₂-CH₂)-(C₆-C₁₀)aryl group optionally substituted by at least one halogen, (C₁-C₂)alkyl group and/or (C₁-C₂)alkoxy group.

A preferred group of compounds according to the invention comprises compounds of formula (XIV), wherein R⁶ is H, -CO₂H, -CO-NH₂, or -CO₂R¹⁰, R¹⁰ being a (C₁-C₈)alkyl group, preferably Me.

A preferred group of compounds according to the invention comprises compounds of formula (XIV), wherein R⁶ is H or -CO₂H.

A preferred group of compounds according to the invention comprises compounds of formula (XIV), wherein:
- R is a group -C(=O)-R', R' being a (C₁-C₁₂)alkyl group, preferably a methyl group;
- R² is a -CH₂-(C₃-C₆)cycloalkyl group, preferably a -CH₂-cyclohexyl group;
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NH₂, wherein
   * q is 1, 2, 3 or 4,
   * R^{7a} is a hydrogen atom or a (C₁-C₈)alkyl group;
   * R^{7b} is a hydrogen atom or a (C₁-C₈)alkyl group, and
   * R⁸ is a hydrogen atom or a (C₁-C₈)alkyl group;
   R³ being preferably -CH₂-COOH;
- R⁴ is a (C₁-C₈)alkyl group;
- R⁵ is a -(CH₂)-(C₆-C₁₀)aryl group optionally substituted by at least one halogen, (C₁-C₂)alkyl group and/or (C₁-C₂)alkoxy group, or a -(CH₂-CH₂)-(C₆-C₁₀)aryl group optionally substituted by at least one halogen, (C₁-C₂)alkyl group and/or (C₁-C₂)alkoxy group; and
- R⁶ is H, -CO₂H, -CO-NH₂, or -CO₂R¹⁰, R¹⁰ being a (C₁-C₈)alkyl group, R⁶ being preferably COOH or H.

The present invention also relates to compounds having one of the following formulae:

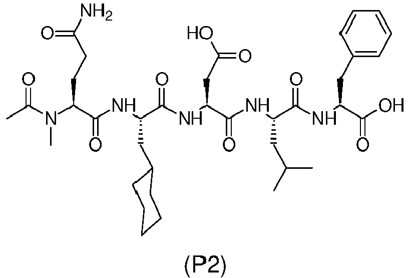

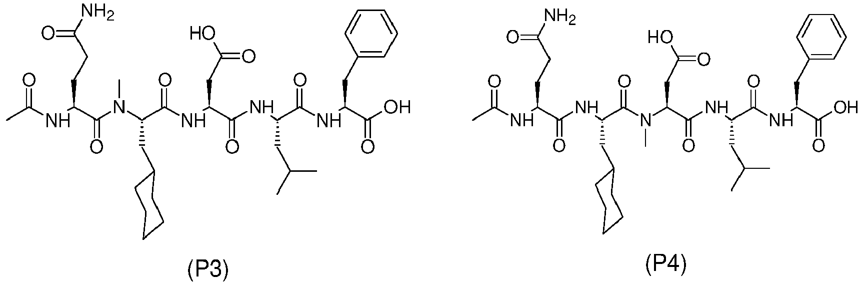

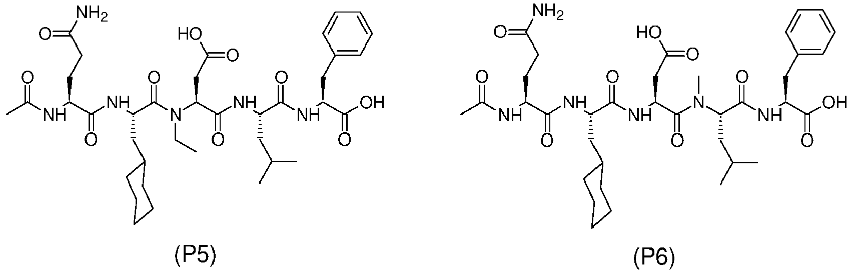

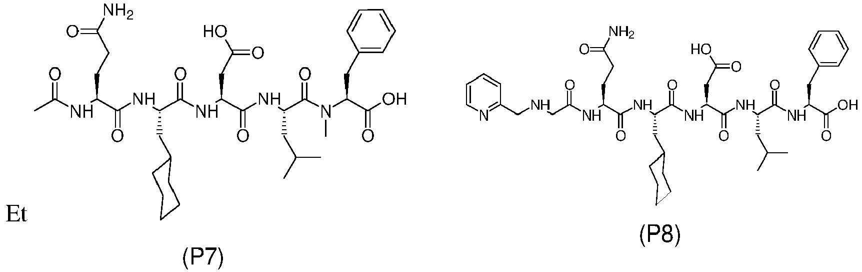

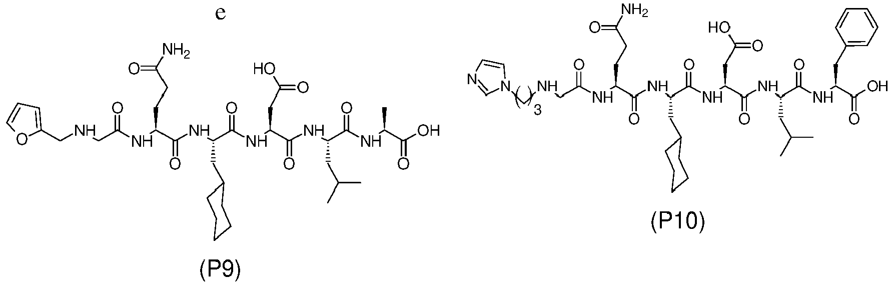

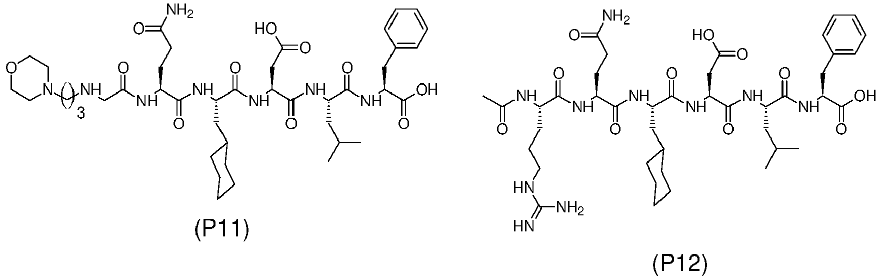

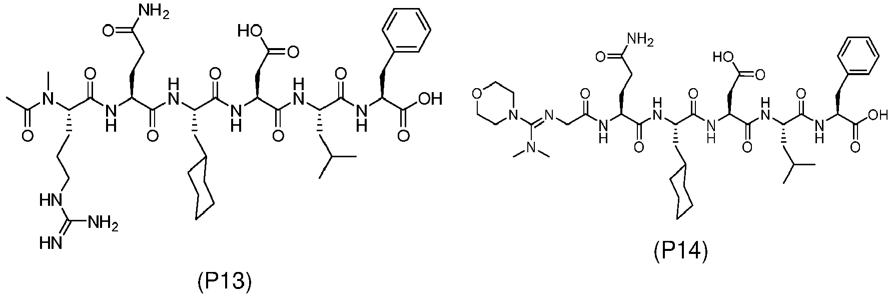

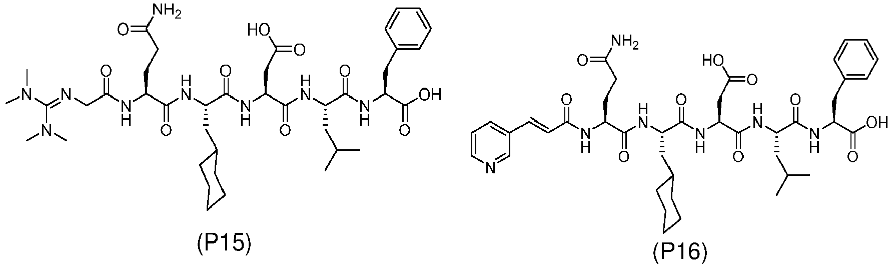

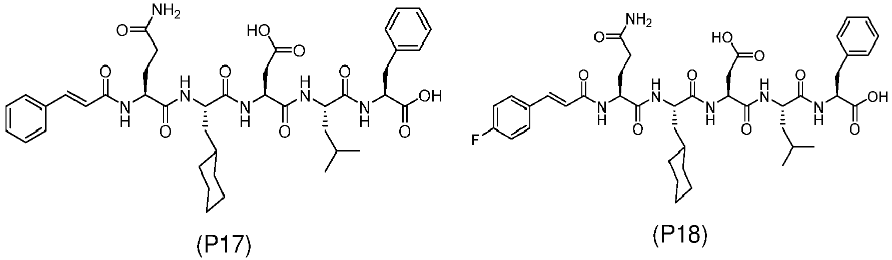

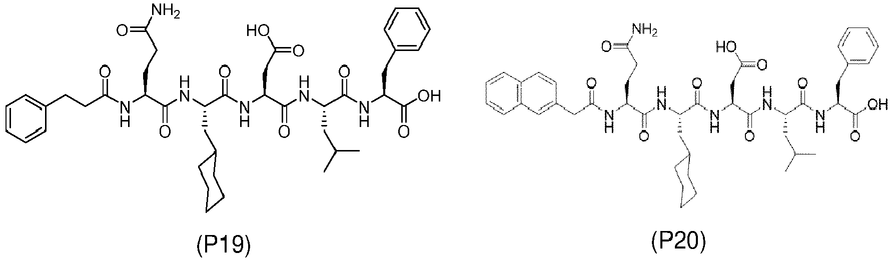

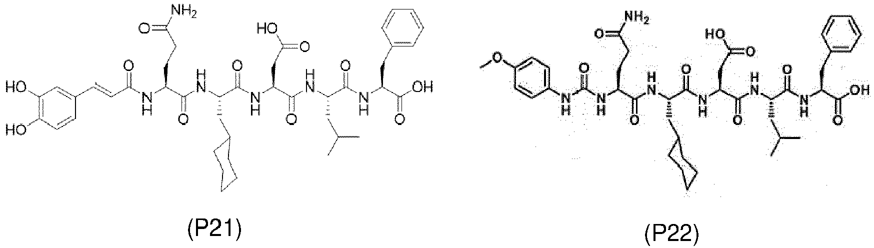

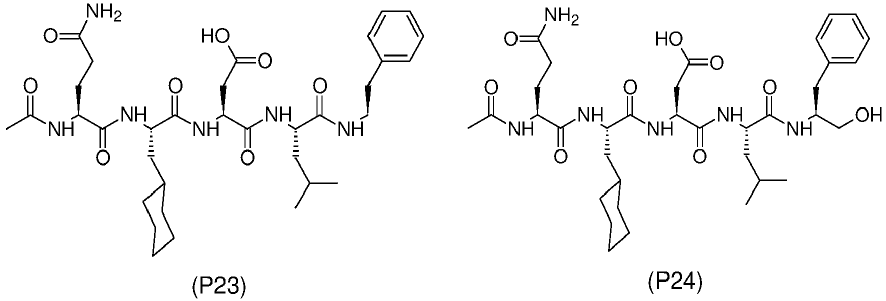

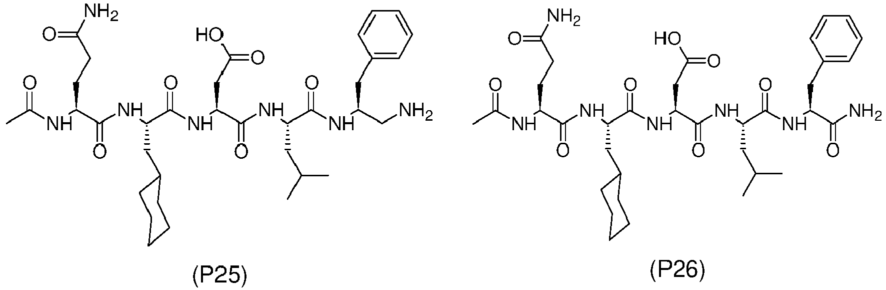

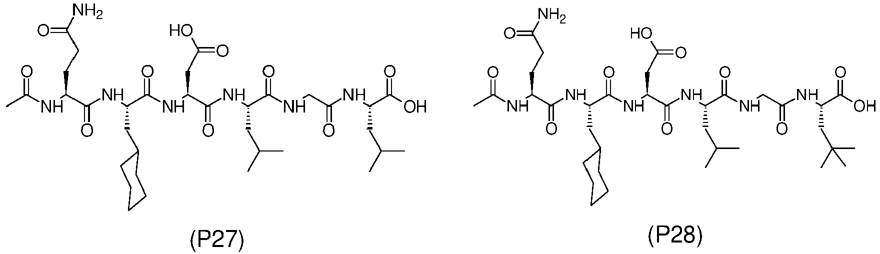

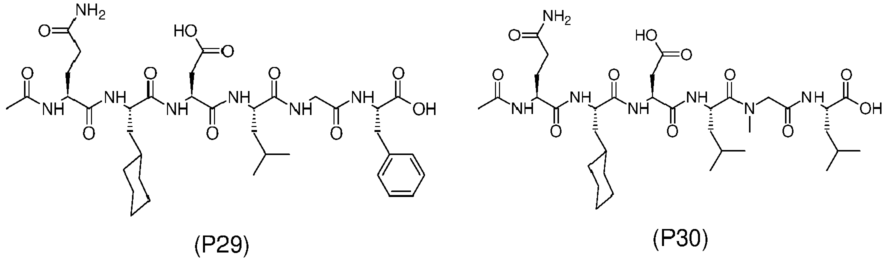

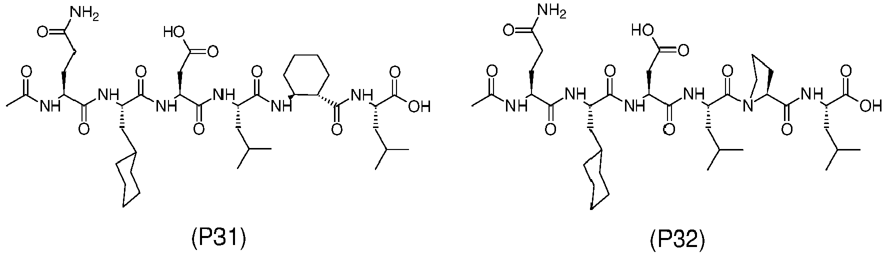

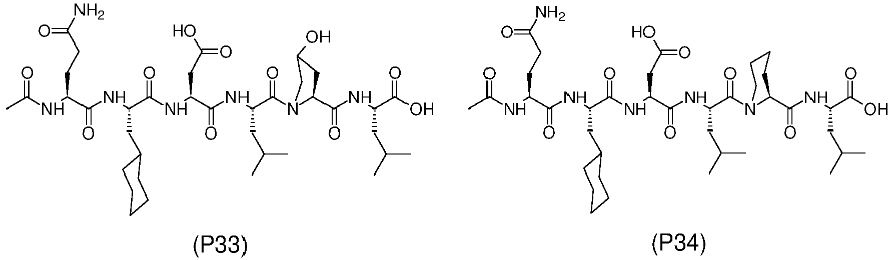

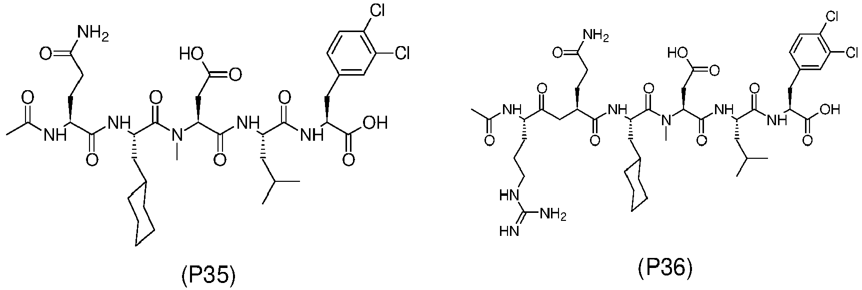

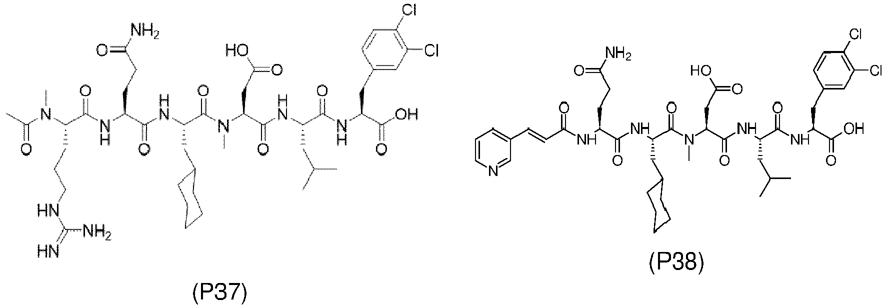

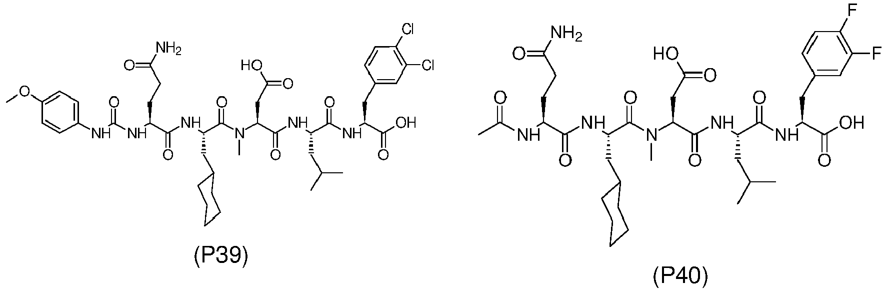

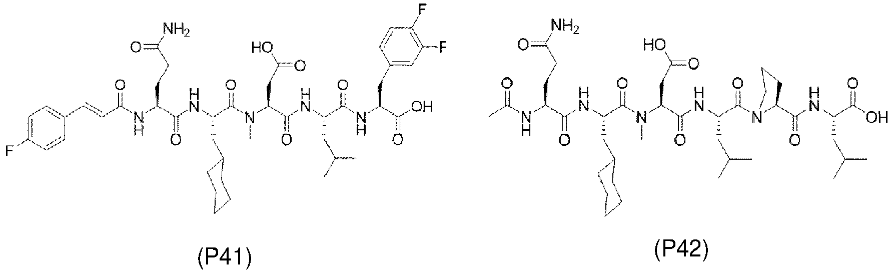

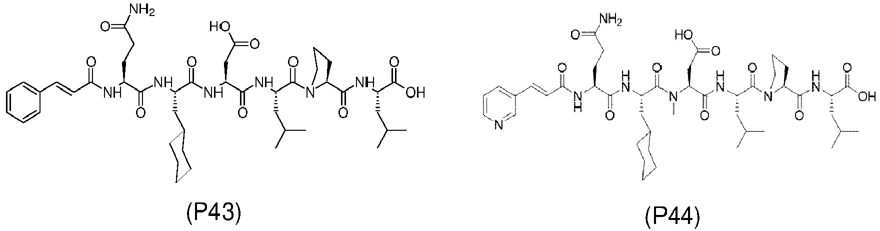

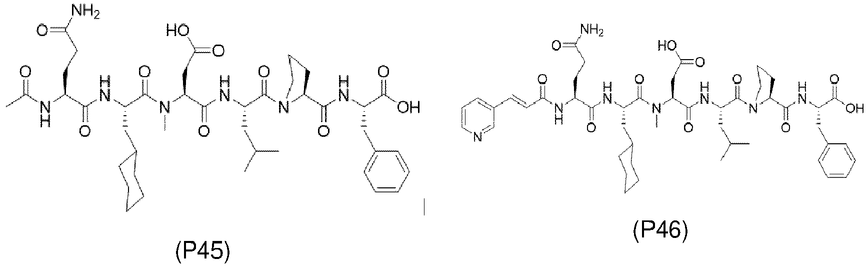

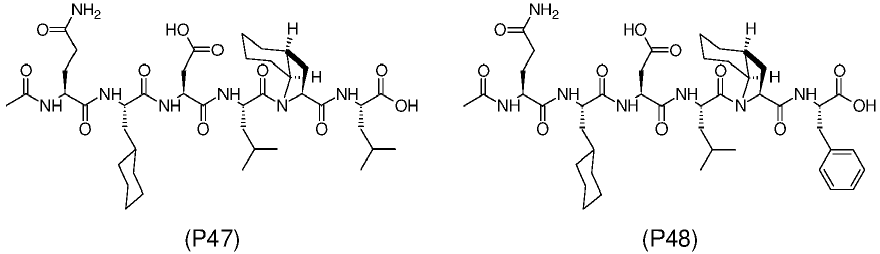

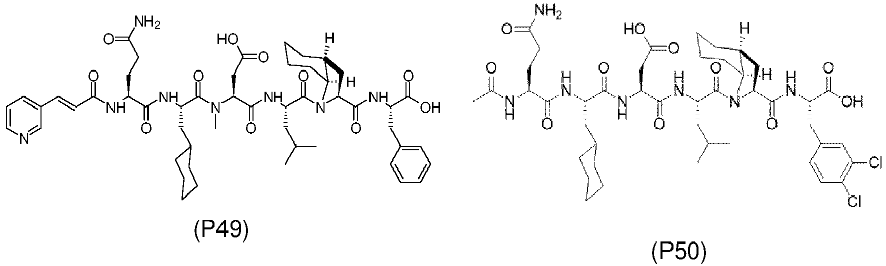

The present invention also relates to the following preferred compounds:

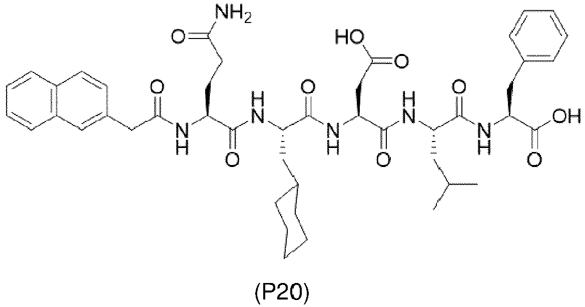

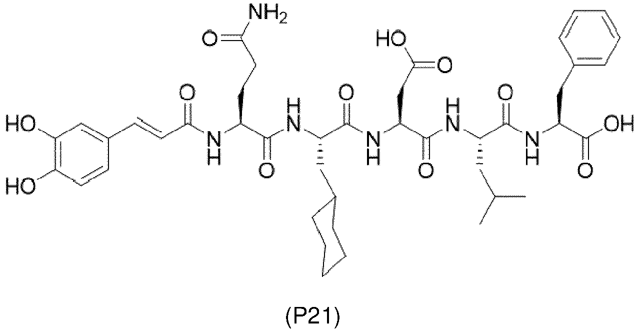

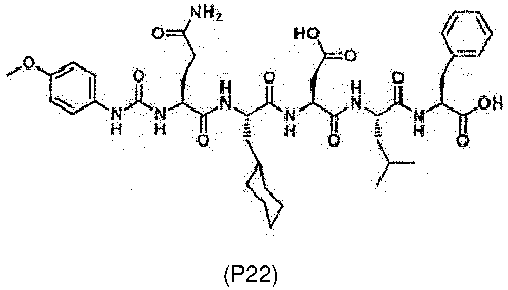

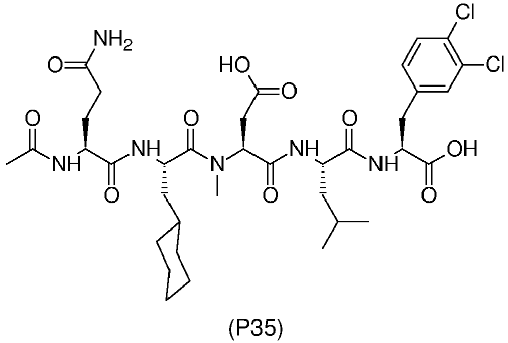

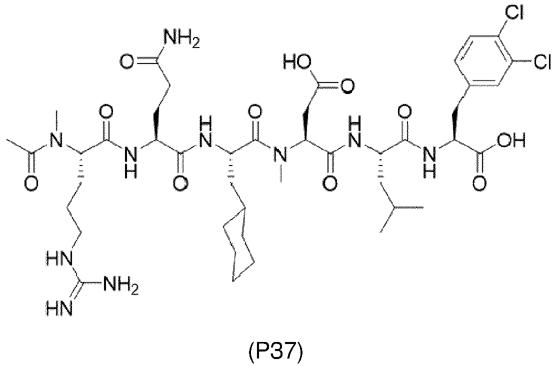

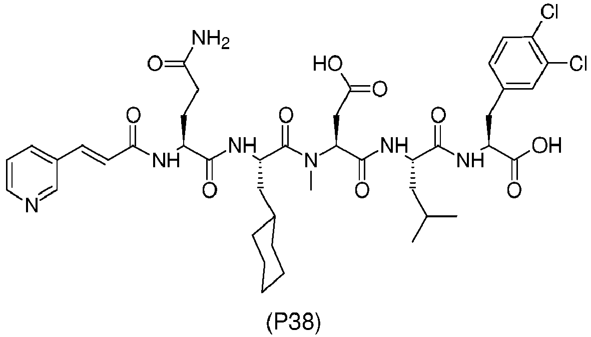

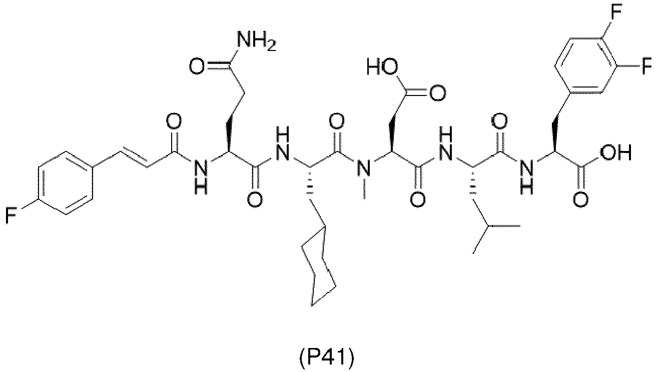

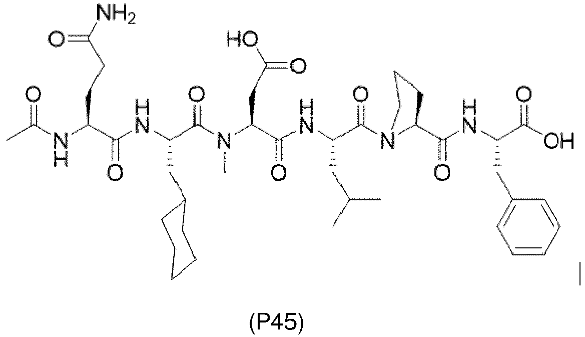

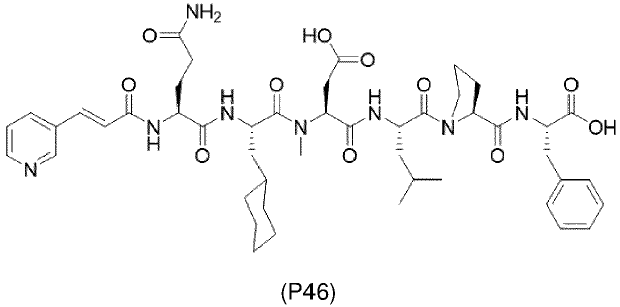

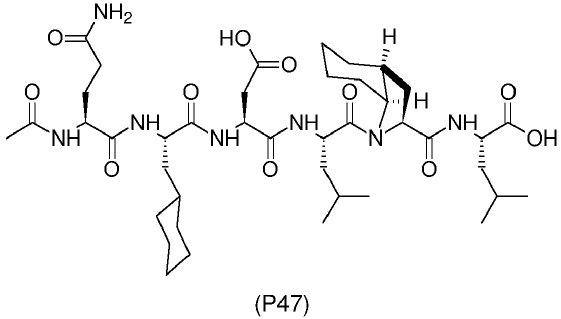

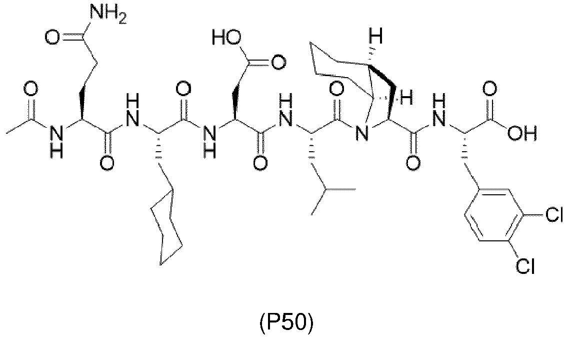

The present invention also relates to chemical derivatives of the compounds of formula (I), (III), (IV) VI), (VII), (VIII), (IX), (X), (XI), (XII), (XIII) or (XIV), comprising at least one addition of chemical, aminopeptidic or nucleic compound, preferentially through a covalent bond, in order to enhance the properties of said compounds, more specifically their stability, affinity or delivery.

The present invention also relates to a medicament comprising a compound of formula (I), (III) or (IV) as defined above.

The present invention also relates to a medicament comprising a compound of formula (V), (VI), (VII), (VIII), (IX), (X), (XI), (XII), (XIII) or (XIV) as defined above.

The present invention also relates to a pharmaceutical composition comprising a compound as defined above, preferably a compound having the formula (I), (III) or (IV) as defined above, in association with a pharmaceutically acceptable vehicle.

The present invention also relates to a pharmaceutical composition comprising a compound of formula (V), (VI), (VII), (VIII), (IX), (X), (XI), (XII), (XIII) or (XIV) as defined above, in association with a pharmaceutically acceptable vehicle.

While it is possible for the compounds of the invention to be administered alone it is preferred to present them as pharmaceutical compositions. The pharmaceutical compositions, both for veterinary and for human use, useful according to the present invention comprise at least one compound having formula (I), (III) or (IV) as above defined, together with one or more pharmaceutically acceptable carriers and optionally other therapeutic ingredients.

In certain preferred embodiments, active ingredients necessary in combination therapy may be combined in a single pharmaceutical composition for simultaneous administration.

As used herein, the term "pharmaceutically acceptable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectables either as liquid solutions or suspensions; however, solid forms suitable for solution, or suspensions, in liquid prior to use can also be prepared. The preparation can also be emulsified. In particular, the pharmaceutical compositions may be formulated in solid dosage form, for example capsules, tablets, pills, powders, dragees or granules.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The pharmaceutical compositions can be administered in a suitable formulation to humans and animals by topical or systemic administration, including oral, rectal, nasal, buccal, ocular, sublingual, transdermal, rectal, topical, vaginal, parenteral (including subcutaneous, intra-arterial, intramuscular, intravenous, intradermal, intrathecal and epidural), intracisternal and intraperitoneal. It will be appreciated that the preferred route may vary with for example the condition of the recipient.

The formulations can be prepared in unit dosage form by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Total daily dose of the compounds of the invention administered to a subject in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

The present invention also relates to the compound of formula (I), (III) or (IV) as defined above for use as an antibacterial agent.

The present invention also relates to a compound of formula (I), (III) or (IV) as defined above for use for the treatment of bacterial infections.

According to an embodiment, the bacterial infections are caused by Gram negative pathogens. Preferably, said bacterial infections are caused by bacteria belonging to the following bacteria families: Enterobacteriaceae, Pseudomonadaceae, Burkholderiales, Neisseriaceae, Campylobacterales, and Francisellaceae.

According to an embodiment, said bacterial infections are caused by bacteria selected from the group consisting of: bacteria from the genus *Escherichia* sp, in particular *E. coli;* bacteria from the genus *Klebselia* sp, in particular *K. pneumoniae;* bacteria from the genus *Shigella* sp., in particular *S*. *sonnei, S*. *dysenteriae,* and *S*. *flexneri;* bacteria from the genus *Salmonella* sp., in particular *S*. *enterica, S. typhi,* and *S*. *parathyphi;* and bacteria from the genus *Yersinia* sp., in particular *Y. enterocolitica* and *Y. pestis.*

According to an embodiment, said bacterial infections are caused by bacteria selected from the group consisting of: bacteria from the genus *Acinetobacter* sp, in particular *A.baumannii;* and bacteria from the genus *Pseudomonas* sp., in particular *P. aeruginosa.*

According to an embodiment, said bacterial infections are caused by bacteria selected from the group consisting of: bacteria from the genus *Burkholderia* sp, in particular *Burkholderia cepacia.*

According to an embodiment, said bacterial infections are caused by bacteria selected from the group consisting of: bacteria from the genus *Neisseria* sp, in particular *N.gonorrhoeae* and *N. meningitidis.*

According to an embodiment, said bacterial infections are caused by bacteria selected from the group consisting of: bacteria from the genus *Campylobacter* sp, in particular *C. jejuni.*

According to an embodiment, said bacterial infections are caused by bacteria selected from the group consisting of: bacteria from the genus *Francisella* sp., in particular *Francisella tularensis.*

According to an embodiment, the infections according to the invention are chosen from infections caused by multi-drug resistant (MDR) bacteria, extensively drug-resistant (XDR) bacteria or pandrug-resistant (PDR) bacteria, derived from the above bacteria.

According to an embodiment, the infections according to the invention are chosen from infections caused by antibiotic-resistant bacteria, in particular with carbepenem resistance, cephalosporin resistance, or fluoroquinolone resistance. Such antibiotic-resistant bacteria are in particular mentioned in the WHO website (http://www.who.int/medicines/publications/WHO-PPL-Short_Summary_25Feb-ET_NM_WHO.pdf?ua=1).

According to an embodiment, the bacterial infections are selected from the group consisting of the following infections: respiratory infections, stomach infections, gastrointestinal infections, blood infections, skin infections, bladder infections, kidney infections, urinary tract infections, ear infections, eye infections, and meningial infections. A skin infection may include an infection of a mucosal membrane, such as the oral cavity, oesophagus or eye, e.g. cornea.

In the context of the invention, the term "treating" or "treatment", as used herein, means reversing, alleviating, inhibiting the progress of, or preventing the disorder or condition to which such term applies, or one or more symptoms of such disorder or condition.

### EXAMPLES

Hereafter are described the synthesis, the binding affinity and the thermodynamic parameters of peptides of formula (I) as defined above, corresponding to new series of peptides derived from the previously reported lead sequence Ac-Gln-Cha-Asp-Leu-Phe-OH (peptide **1,** Cha = β-Cyclohexyl-L-alanine). Guided by X-ray structure analysis, various modifications were introduced at specific positions to i) improve the affinity of the peptide to the *^{Ec}*SC, ii) gain understanding on the structure-activity relationship (SAR), and iii) develop a panel of high-affinity inhibitors displaying favorable physicochemical properties.

### PEPTIDES CHEMICAL SYNTHESIS

*N*-(9-fluorenyl)methoxycarbonyl- (Fmoc-) protected amino acids were purchased from Sigma Aldrich, Iris Biotech, or Fluorochem. The wang, rink amide and 2-chlorotrityl chloride resins were purchased from Novabiochem, Sigma Aldrich or Fluorochem. 1,8-Diazabicyclo[5.4.0]undéc-7-ene (DBU), N-methylpyridone (NMP), pyridine, piperidine, o-nitrobenzenesulfonyl chloride (*o*-NBS-Cl), triphenylphosphine, diisopropyl azodicarboxylate (DIAD), 2-mercaptoethanol, acetic anhydride, triisopropylsilane (TIS), trifluoroacetic acid (TFA) cinnamic acid, 4-fluorocinnamic acid and *trans*-3-(3-pyridyl)acrylic acid were purchased from Sigma Aldrich. N-Diisopropylethylamine (DIEA) was purchased from TCI. *Sym*-collidine was purchased from Acros Organic. 4-dimethylaminopyridine (DMAP) and Oxyma were purchased from Fluorochem. N,N'-Diisopropylcarbodiimide (DIC) was purchased from Iris Biotech.

### Anchoring step

Peptides were synthesized with solid-phase methods by stepwise using Fmoc chemistry and CEM DiscoverBio microwaves. Assembly of the protected peptide chains was carried out on a 0.1 or 0.07 mmol scale starting from either Fmoc-Leu-Wang resin, Fmoc-Phe-Wang resin, Wang resin, rink amide resin or 2-chlorotrityl chloride resin. The resins were swollen for 15 min in CH₂Cl₂/DMF mixture in polypropylene syringes equipped with Teflon filter.

### Attachment of the first amino acid on Wang resin.

In a round-bottom flask, 10.0 equiv of Fmoc-amino acid (relative to resin loading) were dissolved in dry CH₂Cl₂ under argon atmosphere. A small amount of dimethylformamide (DMF) may be needed to achieve complete dissolution. The reaction mixture was cooled to 0°C and 5.0 equiv (relative to resin loading) of DIC in dry CH₂Cl₂ was slowly added to the amino acid solution. The mixture was stirred for 20 min at 0°C and CH₂Cl₂ was evaporated under reduced pressure. The residue was dissolved in a minimum of DMF and added to the resin suspension followed by 0.1 equiv (relative to resin loading) of DMAP. The suspension was shaken at room temperature for 1 h.

### Attachment of the first amino acid on 2-chlorotrityl chloride resin

### - Attachment of an alcohol (for P24)

The resin was swollen in a syringe for 15 min in CH₂Cl₂/DMF mixture. 3 equiv of alcohol (relative to the resin) were dissolved in 2 mL of DMF. Then, 6 equiv of pyridine (relative to the resin) were added to the solution of containing the alcohol. The solvent containing the resin was removed and the solution with the alcohol was added in the syringe. The suspension was shaken for 24 h at room temperature. The solution was removed from the syringe. A solution of CH₂Cl₂/MeOH/ DIEA: 3.4 mL/0.4 mL/0.2 mL was added to the syringe containing the resin. The suspension was shaken for 30 min at room temperature. The solution was removed from the syringe and the resin was washed with DMF and CH₂Cl₂.

### - Attachment of an amine (for P25)

The resin was swollen in a syringe for 15 min in CH₂Cl₂/DMF mixture. 1.2 equiv of amine (relative to the resin) were dissolved in 3 mL of DMF. Then, 5 equiv of DIEA (relative to the resin) was added to the solution of alcohol. The solvent of the resin was removed and the solution of alcohol was added in the syringe. The suspension was shaken 24 h at room temperature. The solution was removed from the syringe. A solution of CH₂Cl₂/MeOH/DIEA (3.4 mL / 0.4 mL / 0.2 mL) was added to the syringe containing the resin. The suspension was shaken 30 min at room temperature. The solution was removed from the syringe and the resin was washed with DMF and CH₂Cl₂.

### Fmoc deprotection

Fmoc removal was carried out with 4 mL of a DMF/Piperidine (8:2 in v/v) solution and the reaction was performed under microwave irradiation (75 °C, 50 W, 5 min). The resin was filtered and washed with DMF (x 2) and CH₂Cl₂ (x 2).

### General procedure for coupling amino acid building block (natural and unnatural), cinnamic acid, 4-fluorocinnamic acid and trans-3-(3-pyridyl)acrylic acid

A solution of activated Fmoc-amino acid (or cinnamic acid, 4-fluorocinnamic acid, trans-3-(3-pyridyl)acrylic acid) was prepared with Fmoc amino acid (4 equiv) in DMF (2 mL); then Oxyma (4 equiv) and DIC (4 equiv) were successively added and the mixture was introduced into a reaction vessel containing the resin. The coupling reaction was performed under microwave irradiation (75 °C, 30 W, 10 min) and the time was doubled for *N*-alkylated residues. The resin was filtered and then washed with DMF (x 2) and CH₂Cl₂ (x 2).

### General procedure for peptide bond N-alkylation (peptides P2, P3, P6, and P40)

### - o-NBS protection.

After Fmoc deprotection, the resin was washed three times with *N-*methylpyridone (NMP). A solution of *o*-NBS-Cl (4 equiv) in NMP (2 mL) was prepared and then sym-collidine (10 equiv) was added. The resin was treated with the solution for 20 min at room temperature with automatic shaking. After, the resin was filtered and washed with NMP (x 3).

### - N-methylation, DBU-mediated method.

A solution of DBU (3 equiv) in NMP (1 mL) was used to treat the resin for 10 min at room temperature. Then, dimethyl sulfate DMS (10 equiv) in NMP (1 mL) was added to the resin and the mixture was shaken at room temperature for 5 min more. After the resin was filtered and washed with NMP (x 3).

### - Mitsunobu reaction-mediated method.

The resin was rinsed with dry THF because the reaction was performed in this new solvent. Then, triphenylphosphine (5 equiv) was dissolved in dry THF (1 mL) and the appropriate alcohol (10 equiv) was added to the solution. The resin was treated with the mixture for 5 min at room temperature and by vigorous shaking. A solution of DIAD (5 equiv) in dry THF (1 mL) was added to the resin in a portionwise manner, taking 250 µl of the solution each time, and allowed to react with automatic shaking for 5 min each time. After the resin was filtered and washed with THF (x 2) followed by NMP (x 3).

### - o-NBS deprotection

The Nosyl-protecting group was eliminated with a solution of 2-mercaptoethanol (10 equiv) and DBU (5 equiv) in NMP (2 mL) and the resin was treated with at room temperature for 10 min with automatic shaking. After the resin was filtered and washed with NMP (x 3).

Each step, protection, alkylation and deprotection, were successively repeated twice using the same conditions described below.

### General procedure for acetylation

A solution of acetic anhydride (10 equiv) in DMF (2 mL) in the presence of DIEA (10 equiv) was introduced in a reaction vessel containing the resin. The coupling reaction was carried out under microwave irradiation (50°C, 30 W, 15 min). The resin was filtered and then washed with DMF (x 2) and DCM (x 2).

### Cleavage of the peptides from the resin

At the end of the elongation of the peptide chain, the resin was washed with CH₂Cl₂ and dried under high vacuum. Then, the cocktail cleavage corresponding to TFA/H₂O/TIS (95:2.5:2.5; 4 mL) was added to the resin. The mixture was gently shaken for 2 h (or 4 h in presence of Arg(Pbf)) and the resulting solution was flushed through a frit. The cleavage cocktail was removed under reduced pressure and the residue was dissolved directly in a mixture of H₂O/MeCN and freeze-dried.

### Peptide purification and characterization

The crudes were purified by semi-preparative RP-HPLC with appropriate gradient and pure fractions were freeze-dried. All peptides were characterized by LCMS ESI₊ mass spectrometry and by analytical C₁₈ RP-HPLC. The given yields are calculated after RP-HPLC semi-preparative purification based on the resin loading.

By implementing the above protocols, the peptides (P1)-(P50) as mentioned above are prepared.

### ACTIVITY OF THE COMPOUNDS

### Methods

### Production and purification of processivity factors.

The procedure has been described previously (Wolff, P.; Oliéric, V.; Briand, J. P.; Chaloin, O.; Dejaegere, A.; Dumas, P.; Ennifar, E.; Guichard, G.; Wagner, J.; Burnouf, D. Y., Structure-Based Design of Short Peptide Ligands Binding onto the E. coli Processivity Ring. J. Med. Chem. 2011, 54 (13), 4627-4637). Briefly, *E.coli* cells were transformed with plasmids pET15b containing the dnaN gene from *E. coli* and were grown in LB at 37°C to OD 0.5, then induced by IPTG (0.1mM) at 28°C overnight. dnaN proteins fractions were first enriched on a Ni-NTA column, eluted with a histidine step (300 mM) and further purified on a Source Q column in buffer containing 20 mM Tris HCl pH 7.5, 0.5 mM EDTA and 10% (v/v) glycerol, using a gradient from 0 to 0.5 M NaCl. After a final ultracentrifugation (45K, 1 h, 20°C), soluble proteins were concentrated on a Centricon 30K (Millipore) in the same buffer and stored at 4°C in 2 M ammonium sulfate. Buffer exchange was performed on Centrikon 10K at 4°C before use and protein quality was assessed by DLS.

### Isothermal titration calorimetry.

ITC experiments were performed using an iTC₂₀₀ or a PEAQ-ITC instrument (Microcal Malvern Panalytical). Peptides (300 or 600 µM) were titrated at 25°C by sequential injections (usually 2 µl each) into an SC solution (30 or 60 µM). Data were corrected for heat of injection by subtracting the signal of the titration of peptides into proteiN-free buffer solution (Hepes 10 mM pH 7.4, NaCl 0.15 M, EDTA 3 mM). Each titration was performed at least twice. Analyses of experimental data were performed following a classical treatment with the AFFINImeter software (https://www.affinimeter.com; S4S, Santiago de Compostela, Spain). In addition, kinetic information was obtained in some cases with kinITC as implemented in the software AFFINImeter.

### Crystallogenesis, X-ray diffraction, data collection and processing.

Crystals of ^{*Ec*w*t*}SC-peptide complexes were obtained by screening the crystallization reagent kit PEG/Ion HT from Hampton Research. 200 nL sitting drops were prepared on plates (Greiner XTL round) by mixing 100 nL of preformed SC/ ligand complexes (protein concentration from 6 to 12 mg/ml; protein/peptide ratio: 1.5) with 100 or 200 nL of crystallization solution, using a Mosquito pipetting station (TTP Labtech). Plates were incubated at 20°C. Crystals were obtained in several crystallization conditions and were frozen in liquid ethane. The data were collected at the X06DA PXIII beamline at the Swiss Light Source (Villigen, Switzerland), equipped with a PILATUS 2M detector and the multiaxis PRIGo goniometer (Waltersperger, S.; Olieric, V.; Pradervand, C.; Glettig, W.; Salathe, M.; Fuchs, M. R.; Curtin, A.; Wang, X.; Ebner, S.; Panepucci, E.; Weinert, T.; Schulze-Briese, C.; Wang, M., PRIGo: a new multi-axis goniometer for macromolecular crystallography. J. Synchrotron Radiat. 2015, 22 (4), 895-900).The wavelength was 1.00 Å, with a flux at full transmission of 4·10¹¹ photons·s⁻¹. The data were collected with two rotations of 360° at χ=0° and χ=30° (0.2° and 0.1s per frame). The crystal-to-detector distance was 300 mm. The data were processed and merged using the software autoPROC version 1.0.5 and STARANISO (Cambridge, United Kingdom: Global Phasing Ltd). The structures were solved by molecular replacement using the software MOLREP version 11.4.04 and search models 1OK7. Refinement was performed with BUSTER version 2.10.3 (Cambridge, United Kingdom: Global Phasing Ltd) and phenix.refine version 1.10.1-2155, with NCS constraints for all structures and TLS for higher resolution *E. coli* structures. 5% of reflections were set aside for the *R*_{free} test set.

### RESULTS

### Lessons from the interaction between peptide 1 and ^{Ec}SC.

The interaction between pentapeptide 1 and *^{Ec}*SC was analyzed by ITC and gave a *K*_{d} of 194 nM (ΔG of -9.2 kcal mol⁻¹) **(Table 1),** in good agreement with the *K*_{d} values previously determined by SPR (170 nM)(Wolff, P.; Oliéric, V.; Briand, J. P.; Chaloin, O.; Dejaegere, A.; Dumas, P.; Ennifar, E.; Guichard, G.; Wagner, J.; Burnouf, D. Y., Structure-Based Design of Short Peptide Ligands Binding onto the E. coli Processivity Ring. J. Med. Chem. 2011, 54 (13), 4627-4637).

**Table 1. Structures, dissociation constants, thermodynamic and kinetic parameters (determined by ITC at 298.15 K) of the interaction with of ^{Ec}SC with different N-alkylated analogs of peptide 1.**

| | | | | | | | | | |
|---|---|---|---|---|---|---|---|---|---|
| 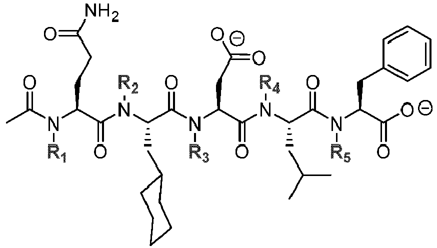 | | | | | | | | | |

| Compd | R₁ | R₂ | R₃ | R₄ | R₅ | *K*_{d} (nM) | ΔG (kcal/mol) | *kₒₙ* (M⁻¹.s⁻¹) | *k_{off}* (s) |
|---|---|---|---|---|---|---|---|---|---|
| **P1** | H | H | H | H | H | 194 (± 58) | -9.2 (± 0.2) | 4 (± 1.3) 10⁴ | 7.4 (±2.9) 10⁻³ |
| **P2** | Me | H | H | H | H | > 1000 | -7.7 (± 0.2) | 2.1 (± 1.9) 10⁴ | 4.6 (± 1.5) 10⁻² |
| **P3** | H | Me | H | H | H | > 1000 | (± 0.1) | nd | nd |
| **P4** | H | H | Me | H | H | 114 (± 31) | -9.5 (± 0.1) | 2,1 (± 1.3) 10⁵ | 1,9 (±0.8) 10⁻² |
| **P5** | H | H | Et | H | H | 523 (± 65) | -8.57 (± 0.07) | 2,88 (± 0.6) 10⁴ | 1,29 (± 0.2) 10⁻² |
| **P6** | H | H | H | Me | H | > 1000 | ₍± 0.2) | nd | nd |
| **P7** | H | H | H | H | Me | > 1000 | -7.1 (± 0.1) | 1 (± 0.6) 10⁴ | 5.4 (± 0.5) 10⁻² |

| | | | | | | | | | |
|---|---|---|---|---|---|---|---|---|---|
| nd: not detected | | | | | | | | | |

Additional information about the kinetics of the interaction of **1** with SC was obtained by treatment of the ITC injection curves using the kinITC program (*k*ₒₙ = 4 10⁴ M⁻¹.s⁻¹ and *k*_{off} = 7.4 10⁻³ s). These values were used as a reference for head-to-head comparison with all analogs reported in this application. The structure of the complex between **1** and *^{Ec}*SC, which was recently reported (PDB ID: 6FVL) was used to guide further optimizations of peptide **1** (André, C.; Martiel, I.; Wolff, P.; Landolfo, M.; Lorber, B.; Silva da Veiga, C.; Dejaegere, A.; Dumas, P.; Guichard, G.; Oliéric, V.; Wagner, J.; Burnouf, D. Y., Interaction of a Model Peptide on Gram Negative and Gram Positive Bacterial Sliding Clamps. ACS Infect. Dis. 2019, 5 (6), 1022-1034).
The peptide is involved in five hydrogen bonds (HBs) which contribute to the binding affinity with the protein. The carboxamide side chain of the Gln residue is engaged in a complex HB network involving a water molecule, the carbonyl of Met362 from the protein and the carbonyl of the second residue of the peptide (Cha), as observed previously. The Gln residue is highly conserved among SC binding sequences and is a crucial residue for the peptide interaction. Several substitutions of the Gln residue including Asn, Glu, Ala, Gly were shown previously to lower the affinity. The Asp residue, whose side-chain carboxylate is hydrogen-bonded to His175 was also found to contribute substantially to the interaction, its replacement by an Ala or a Glu being detrimental.
Interestingly, the Asp to Thr substitution does not affect the interaction (K_{d} = 180 (+/-25) nM, ΔG = -9.2 (+/- 0.1) kcal/mol. Finally, three groups of the peptide main chain are in direct contact with the protein surface through HB and may contribute significantly to the binding affinity: the C=O of the acetyl group and the amide NHs of Cha2 and Leu4. However, the other backbone NHs (NH-1, NH-3 and NH-5) could be modulated. To evaluate their contribution and explore the possibility to mask unnecessary NHs, we performed a complete N-methyl scan. This peptide backbone modification is a useful tool to modulate biological activity and selectivity, but can also be used to improve cell permeability and metabolic stability. Based on the X-ray structure, the carboxylate located at the C-terminal position, which is rather pointing towards the solvent, does not seem to interact with the protein surface. Therefore, it was replaced by a non-anionic hydrophilic group like amide, alcohol or amine to remove the negative charge, which may be deleterious for membrane permeation, and investigated whether the binding affinity was affected by these modifications.

In addition to the HB interactions, several hydrophobic contacts are also observed mainly located at the Cha2, Leu4 and Phe5 residues. Among them, the Leu4 and the Phe5 side chains point in a large and deep hydrophobic pocket but the cavity does not seem to be fully occupied by the combination of isobutyl and benzyl side chains, suggesting that bulkier side chains could increase the binding affinity. Therefore, various modulations of the Phe5 were considered here to better occupy the hydrophobic pocket. Moreover, extension of the N-terminus of the pentapeptide with various molecular scaffolds may offer opportunities to create new interactions with the protein surface and improve the binding affinity and thus were also evaluated.

All newly synthesized peptides were tested for their interaction with *^{Ec}*SC by ITC at 25°C. As a general rule, all thermodynamic profiles reveal an exothermic interaction, and a non-favorable entropic component. The resulting negative Gibb's free energies characterize a spontaneous interaction. The analysis of raw data by the Affinimeter^{®} software also gave access to kinetics rate constant of the interaction.

### Impact of sequential backbone N-methylation/-ethylation.

The influence of N-methylation of the peptide was investigated systematically on each amide of the backbone of the reference peptide **1** (Table 1).

Remarkably, N-methylation of Asp3 (compound P4) retains an affinity similar to that of **1.** The entropic factor is slightly but significantly weaker for P4, suggesting that the N3-methylation attenuate the entropic penalty of the peptide/SC interaction observed for **1.** Noteworthy, the *k*ₒₙ value measured for P4 is 5-fold higher than for P1, while *k_{off}* values are 2.5 fold higher (Table 1).
The structure of the *^{Ec}*SC-P4 complex has been solved at 2.9 Å. The asymmetric unit contains four rings and eight peptides that superimpose nicely with peptide **1.** Within the asymmetric unit, the N3-methyl groups sample slightly different orientations away from the SC surface. Additionally, a packing-related crystallographic artifact was observed where two peptides, stacked in an opposite orientation, define a hydrophobic pocket lined by the two Cha residues and the two N3-methyl groups.

### Effects of modifications at the N-terminal position.

Here, the influence of various groups that would extend further from the N-terminus (peptides P8 to P22; Table 2) but keeping the carbonyl at this position were analyzed.

**Table 2. Structures, dissociation constants thermodynamic and kinetic parameters determined by ITC of analogs of 1 modified at the N-terminal position.**

| | | | | | |
|---|---|---|---|---|---|
| 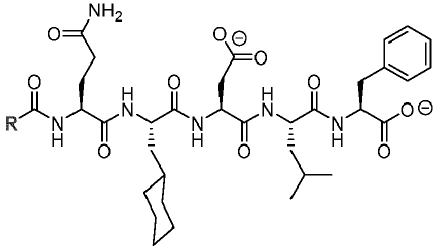 | | | | | |

| Compd | R | *K*_{d} (nM) | ΔG (kcal/mol) | *kₒₙ* (M⁻¹.s⁻¹) | *k_{off}* (s) |
|---|---|---|---|---|---|
| **P8*** | 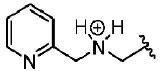 | 172 (± 29) | -9.2 (± 0.1) | 3,4 (± 0.9) 10⁴ | 5,7 (± 0.9) 10⁻³ |
| **P9** | 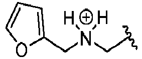 | 171 (± 0.3) | -9.2 (± 0.1) | 4.8 (± 1.6) 10⁴ | 8.6 (± 4.7) 10⁻³ |
| **P10** | 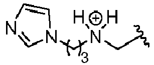 | 312 (± 78) | -8.9 (± 0.1) | 4.2 (± 3.2) 10⁴ | 1,1 (± 0.6) 10⁻² |
| **P11** | 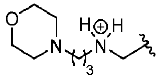 | 796 (± 12) | -8.3 (± 0,01) | 1.8 (± 0.1) 10⁴ | 1.4 (± 0.1) 10⁻² |
| **P12** | 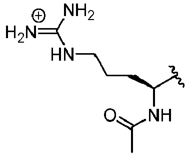 | 498 (± 66) | -8.60 (± 0.08) | 3.7 (± 1.3) 10⁴ | 1.9 (± 0.9) 10⁻² |
| **P13** | 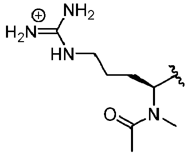 | 2130 (± 360) | -7.7 (± 0.1) | 2.1 (± 1.4) 10⁴ | 2.9 (± 1.1) 10⁻² |
| **P14** | 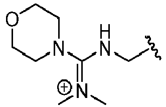 | 276 (± 71) | -9.0 (± 0.1) | 3,4 (± 0.6) 10⁴ | 1 (± 0.1) 10⁻² |
| **P15** | 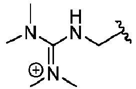 | 353 (± 70) | -8.7 (± 0.1) | 3,4 (± 1.4) 10⁴ | 1,1 (± 0.4) 10⁻² |
| **P16** | 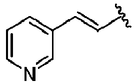 | 156 (± 42) | -9.3 (± 0.2) | 4,6 (± 1.5) 10⁴ | 7,4 (± 3.9) 10⁻³ |
| **P17** | 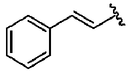 | 129 (± 43) | -9.4 (± 0.2) | 4,6 (± 1.0) 10⁴ | 6,5 (± 2) 10⁻³ |
| **P18*** | 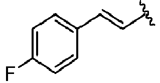 | 144 (± 38) | -9.3 (± 0.2) | 4,6 10⁴ (± 1.4 10⁴) | 6,3 (± 1.2) 10⁻³ |
| **19** | 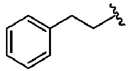 | 123 (± 11) | -9.1 (± 0.05) | 5,5 (± 1.3) 10⁴ | 6,7 (± 1) 10⁻³ |
| **P20*** | 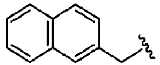 | 89 (± 21) | -9.6 (± 0.1) | 6,5 (± 1.3) 10⁴ | 5,71 (± 0.7) 10⁻³ |
| **P21*** | 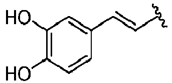 | 99 (± 14) | -9.5 (± 0.1) | 4,6 (± 1.3) 10⁴ | 4,7 (± 1.9) 10⁻³ |
| **P22*** | 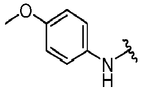 | 73 (± 24) | -9.8 (± 0.2) | 4,0 (± 0.4) 10⁴ | 2,8 (± 0.8) 10⁻³ |

| | | | | | |
|---|---|---|---|---|---|
| * X-ray structures were obtained from protein-ligand co-crystals. | | | | | |

First, the inventors examined the introduction of various basic N-alkyl glycyl derivatives with aromatic substituents including pyridine, furan and imidazole groups (Table 2, compounds P8-P10) but these modifications did not provide any affinity gain compared to **1.** The addition of a Arg residue (peptide P12) does not improve the affinity, while an additional N1-methyl modification (peptide P13) strongly reduces the interaction. For both peptides, this low affinity is related to a higher *k_{off},* respectively 2.5 and 4 fold as compared to **1** (Table 2). Guanidine bearing substituents (compounds P14 and P15) were tolerated but slightly lower affinities were observed compared to that of peptide **1.**

In contrast, the shorter and less hindered 3-pyridinacryloyl termination (peptide P16) led to an affinity equivalent to that of peptide **1.** We extended this series to other 3-arylacryloyl groups (peptides P17-P21). Whereas similar affinities were observed with the cinnamoyl (peptide P17) and 4-fluorocinnamoyl (peptide P18) derivatives, introduction of a catechol (peptide P21) or a 2-naphtyl group (peptide P20) led to substantial improvements with *K*_{d} values around 90 nM. Shortening further the distance between the aromatic ring and the N-terminal position of the peptide by using a urea linkage (ligand P22) was also well tolerated and gave the tightest binder in this series (K_{d} = 73 nM) (Table 2).

Nor the thermodynamic profile, nor the rate constants of the interaction of P20 with ^{Ec}SC were significantly modified as compared to the control 1 (Table 2). In contrast, modifications introduced in P21 and P22 significantly modify the profiles: the reduction of the entropic factor in P21 may be related to the extension of the catechol moiety into the solvent and its resulting agitation. In contrast, the methoxyphenyl moiety of compound P22 is more constrained on the SC surface, which could account for the increase of the entropic factor.

To better characterize the contribution of these peptide N-terminations to the interaction with the protein surface, the co-crystal structures of peptides P8, P18, P20, P21 and P22 bound to *^{Ec}*SC were determined. In all these structures, the peptide backbones superimpose very well with an average rmsd of 0.199 Å between the five equivalent Cα atoms. Several specific interactions are observed between the N-terminal aryl moieties and the residues of the protein located in the vicinity. For example, both the amide and the nitrogen of the pyridinyl ring in peptide P8 are H-bonded to Arg365 whereas in P18, the fluorophenyl ring forms a T-shaped π-π interaction with Phe278.

In contrast, for peptides P20, P21 and P22, despite a very similar position of the peptide backbone on the binding site, the N-terminal aromatic groups do not superimpose with each other nor form π-π stacking interaction with Phe278. In peptide P20, the naphtyl group contributes to the packing via stacking interactions with the amide of the Arg365 residue of the NCS (non-crystallographic symmetry) related ring. The 3,4-dihydroxyphenyl from P21 is oriented toward the solvent and does not interact with the protein, while the methoxyphenyl moiety of P22 is stabilized by one HB with the Arg365 (2.9 Å) cyclohexyl ring. It is noteworthy that in the case of peptide P20 a salt bridge is formed between the carboxylate from the Asp3 with Arg152 from the protein. This is in contrast to all X-ray structures resolved so far for this series of peptide in which the carboxylate is generally H-bonded to His175. In fact, Arg152 is closer to Asp3 by 1.1 to 1.5 Å because of its stacking with the naphtyl group of the other NCS related peptide. Globally, the interaction with *^{Ec}*SC appears to be quite permissive to substituent variations (size, functions) introduced at the N-terminus of peptide **1.** Interestingly, several aromatic groups introduced at this position led to a significant increase in affinity (peptides P20-P22). Alternatively, some groups, such as those in peptides P16, P18 and P19 do not improve the affinity alone but prove to be of interest when combined with other modifications (see below).

### Variations at the peptide C-terminal position.

With the goal to gain additional insights into the relationship between the sequence and affinity for *^{Ec}*SC as well as to modulate the physicochemical properties of the peptide, the effect of two series of modifications at the C-terminus were examined, namely: i) the replacement of the main chain carboxylate (Table 3, Peptides P23-P26), and ii) the extension of the chain by introduction of a hinge residue (Table 3, Peptides P27-P34), as found in the binding sequence of the polymerase IV (LGL motif).

**Table 3. Structures, dissociation constants and thermodynamic parameters determined by ITC of analogs of the peptide 1 modified at the phenylalanine residue.**

| | | | | | |
|---|---|---|---|---|---|
| 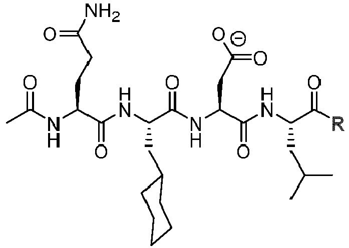 | | | | | |

| Compd | R | K_{d} (nM) | ΔG (kcal/mol) | *kₒₙ* (M⁻¹.s⁻¹) | *k_{off}* (s) |
|---|---|---|---|---|---|
| **P23** | 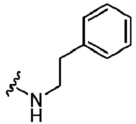 | 922 (± 70) | -8.2 (± 0.05) | 1.9 (± 0.4) 10⁴ | 1.8 (± 0.5) 10⁻² |
| **P24** | 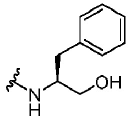 | 751 (± 130) | -8.4 (± 0.1) | 2.6 (± 0.8) 10⁴ | 1.9 (± 0.3) 10⁻² |
| **P25** | 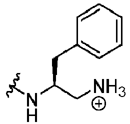 | 877 (± 45) | -8.27 (± 0.03) | 1.2 (± 0.2) 10⁴ | 1.1 (± 0.01) 10⁻² |
| **P26** | 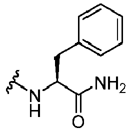 | 268 (± 68) | -9.0 (± 0.1) | 3.3 (± 1.1) 10⁴ | 9.1 (± 4.3) 10⁻³ |
| **P27** | 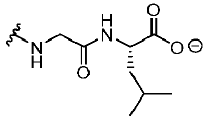 | 682 (± 154) | -8.4 (± 0.1) | 2.6 (± 0.7) 10⁴ | 1.7 (± 0.3) 10⁻² |
| **P28** | 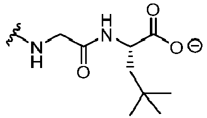 | 2690 (± 290) | -7.60 (± 0.06) | 1.1 (± 0.3) 10⁴ | 3.0 (± 0.4) 10-2 |
| **P29** | 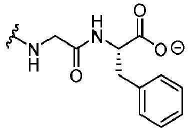 | 313 (± 50) | -8.9 (± 0.1) | 3.2 (± 0.7) 10⁴ | 1.1 (± 0.3) 10⁻² |
| **P30** | 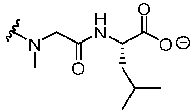 | 583 (± 159) | -8.5 (± 0.2) | 4.0 (± 2.0) 10⁴ | 2.1 (± 0.5) 10⁻² |
| **P31** | 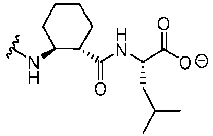 | 4070 (± 1520) | -7.4 (± 0.2) | 7.4 (± 2.5) 10³ | 2.9 (± 1.1) 10⁻² |
| **P32** | 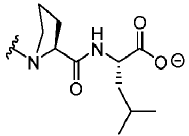 | 182 (± 79) | -9.2 (± 0.2) | 5.7 (± 1.8) 10⁴ | .1 (± 0.6) 10⁻² |
| **P33** | 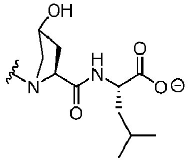 | 369 (± 93) | -8.8 (± 0.2) | 4.4 (± 1.1) 10⁴ | 1.7 (± 0.7) 10⁻² |
| **P34** | 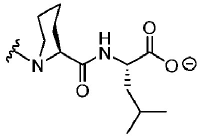 | 243 (± 45) | -9.0 (± 0.1) | 4.3 (± 1.5) 10⁴ | 1.0 (± 0.2) 10⁻² |

A series of peptides in which the carboxylate was removed (P23) or substituted by CH₂OH (P24), CH₂NH₃⁺ (P25), and CONH₂ (P26) was synthesized and tested.

Interestingly, the comparison of thermodynamic profiles for these three peptides shows a strong reduction of the enthalpic and entropic factors amplitudes, notably upon introduction of hydroxyl (P24) or amine (P25) groups. The introduction of an amide group (P26) yield kinetics and thermodynamic parameters similar to that of **1** (Table 3).

The introduction of pyrrolidine (peptide P32) and piperidine (peptide P34) rings and to a lesser degree, hydroxypyrrolidine (peptide P33) led to a significant gain in affinity, as compared to the parent compound P27 (Table 3). The best compound in this series is the proline containing peptide P32, which exhibited a 4-fold lower K_{d}.

### Effect of multiple residue replacements in the pentapeptide series.

The inventors then investigated in a more systematic way whether combinations of beneficial residue modifications can be additive in terms of binding efficacy. N-methylation of the central aspartic acid residue which was found to be neutral for the interaction (Table 1, peptide P4) was systematically combined with other modifications at the C- and N-terminal positions (Table 4).

**Table 4. Structures, dissociation constants and thermodynamic parameters determined by ITC for the peptides combining modifications at different positions.**

| | | | | | | | |
|---|---|---|---|---|---|---|---|
| 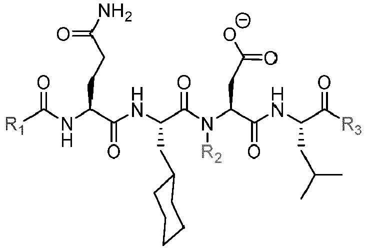 | | | | | | | |

| Compd | R₁ | R₂ | R³ | K_{d} (nM) | ΔG (kcal mol⁻¹) | *kₒₙ* (M⁻¹ s⁻¹) | *k_{off}* (s) |
|---|---|---|---|---|---|---|---|
| **P35*** | CH₃ | CH₃ | 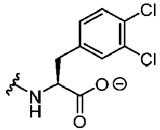 | 41 (± 26) | -10.1 (± 0.3) | 2.7 (± 1.2) 10⁵ | 1.0 (± 0.4) 10⁻² |
| **P36*** | 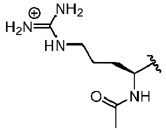 | CH₃ | 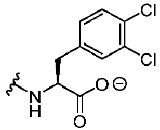 | 278 (± 44) | -8.9 (± 0.1) | 7.6 (± 1.2) 10⁴ | 2.1 (± 0.4) 10⁻² |
| **P37*** | 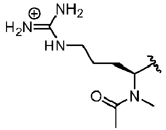 | CH₃ | 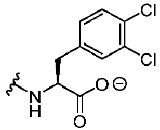 | 92 (± 44) | -9.6 (± 0.2) | 1.1 (± 0.7) 10⁵ | 9.4 (± 4.3) 10⁻³ |
| **P38*** | 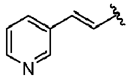 | CH₃ | 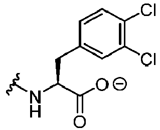 | 46 (± 30) | -10.1 (± 0.4) | 1.1 (± 0.3) 10⁵ | 5.5 (± 0.5) 10⁻³ |
| **P39** | 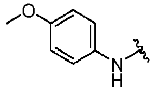 | CH₃ | 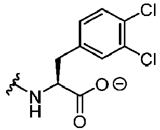 | 266 (± 4) | -9.0 (± 0.01) | 4.2 (± 1.8) 10⁴ | 1.1 (± 0.5) 10⁻³ |
| **P40** | CH₃ | CH₃ | 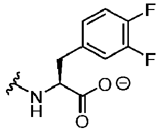 | 148 (± 5) | -9.3 (± 0.02) | 5.6 (± 1.0) 10⁴ | 8.4 (± 1.6) 10⁻³ |
| **P41*** | 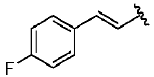 | CH₃ | 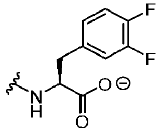 | 99 (± 52) | -9.6 (± 0.3) | 5.1 (± 1) 10⁴ | 5.7 (± 0.2) 10⁻³ |
| **P42** | CH₃ | CH₃ | 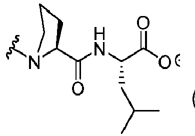 | 263 (± 116) | -9.0 (± 0.2) | 1.0 (± 1.0) 10⁵ | 2.3 (± 2.4) 10⁻² |
| **P43*** | 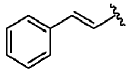 | H | 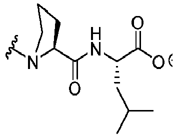 | 132 (± 44) | -9.4 (± 0.2) | 6.0 (± 4.7) 10⁴ | 8.0 (± 5.3) 10⁻³ |
| **P44** | 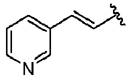 | CH₃ | 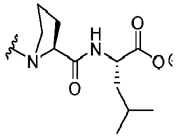 | 177 (± 35) | -9.2 (± 0.1) | 6.3 (± 3.0) 10⁴ | 1.1 (± 0.4) 10⁻² |
| **P45** | CH₃ | CH₃ | 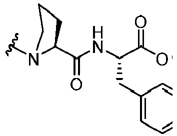 | 81 (± 26) | -9.7 (± 0.2) | 6.0 (± 1.5) 10⁴ | 5.0 (± 1.7) 10⁻³ |
| **P46** | 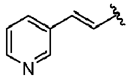 | CH₃ | 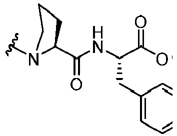 | 36 (± 0.1) | -10.2 (± 0.1) | 1.4 (± 0.5) 10⁵ | 5.2 (± 2.1) 10⁻³ |

| | | | | | | | |
|---|---|---|---|---|---|---|---|
| * X-ray structures were obtained from protein-ligand co-crystal | | | | | | | |

A first series was prepared (compounds P35-P41, Table 4), in which the 3,4-dichlorinated phenylalanine or its 3,4-difluorinated counterpart, both shown to enhance the binding affinity of **1,** were introduced at the C-terminus in combination with several N-terminal groups. The analogue of **1** combining the chlorine atoms on the phenyl ring of Phe5 and the N-methylation of the Asp3, compound P35, was found to display an increased affinity to *^{Ec}*SC (41 nM), as compared to **1,** with no major change in the thermodynamic profile. Additionally, a 7-fold increase of the *kₒₙ* value is measured, as compared to **1** (Table 4). The addition of a N-terminal fluorocinnamoyl group (peptide P41) further increases the affinity of P40 to 99 nM and significantly reduces the *k_{off}* (Table 4), without really affecting the thermodynamic profile, as compared to **1.** Replacement of the Ac by either an Ac-(N-Me)Arg or a pyridinyl acrylamide (peptides P37 and P38 respectively) yielded compounds with comparable affinities, increased on rates and a strong reduction of the ΔH (from 2.4 to 3.5 kcal mol⁻¹) as compared to **1** (Table 4).

Thus the combination of some modifications (P37, P38, P41) appear more efficient in terms of affinity than the single N-ter modifications, as observed in compounds P12, P13 and P16 **(Table 2),** but is not always required to reach a high affinity, as shown in compound P35.

To gain more insights on the contribution of the Arg and the (N-Me)Arg N-terminal residues in peptides P36 and P37 to the affinity, co-crystals of these peptides with the *^{Ec}*SC were grown and the X-ray structures were solved at a resolution of 1.7 Å and 1.6 Å respectively. Interestingly, while for peptide P36, the NH and CO groups from the N-terminal arginine interacts through an HB with the carbonyl and amine groups of the Arg365 from the SC, thus orienting the guanidinium side chain towards the solvent, the N-methylated analog (peptide P37) could not interact in this way anymore. Instead, the N-terminal arginine side chain lies over the SC surface and occupies two distinct positions: one is stabilized through a cation-p interaction with Phe278, while the other position forms a HB with SC Leu366 oxygen atom.

As compared to peptide P35, compound P38 displays the same affinity but shows reduced thermodynamic parameters and also smaller *k_{off}* (Table 4). In order to investigate the basis of these differences, the structure of the *^{Ec}*SC-**38** complex was solved at 1.96 Å. The peptide nicely superimposes with the related P35 ligand (rmsd = 0.43 Å over 332 atoms) and the N-terminal 3-pyridinacryloyl group lies over the SC surface, similarly to peptide **43** N-terminal cinnamoyl group with which it superimposes well. As for P35, the N-terminal carbonyl of P38 establishes an HB with the backbone N atom of Arg365 (3.1 Å) and an additional interaction is established between the N of the pyridine ring and the Arg365 oxygen atom (4.1 Å). These two interactions may account for the higher stability of P38. Noticeably, in the crystal packing, a complex stacking interaction is formed between the 3-pyridinacryloyl groups, the Cha and Arg365 of two peptides of adjacent rings.

### Affinity enhancement using multiple replacements in the hexapeptide series.

The approach of combining multiple positive replacements was extended to the hexapeptide series by preparing several analogues of P32 (peptides P42-P46) (Table 4). Changing the Phe5 (peptide 1) into GlyLeu (peptide P27) at position R3 (Table 3), strongly affects the affinity but reduces the entropic penalty. It is further reduced by replacing the Leu by a Pro residue (peptide P32), which can be accounted for by a better filling of the hydrophobic pocket. In the Pro-Leu series (peptides P42-P44), N-terminal modifications, cinnamoyl (peptide P43) and 3-(3-Pyridyl)acryloyl groups and/or backbone N-methylation at position 3 (peptide P44) did not result in significant improvement of the affinity. In contrast, the combination of proline and phenylalanine at position 5 and 6, respectively (peptide P45) was beneficial in terms of binding with a K_{d} value of 81 nM, as compared to peptide P42 (K_{d} = 263 nM). Additional N-terminal modification with a 3-(3-Pyridyl)acryloyl group (peptide P46) yielded another substantial increase of the affinity (*K*_{d} = 36 nM) and a strong reduction of the entropic factor in comparison to peptide P45 and to cognate peptides P4 (114 nM) and P16 (156 nM), both containing a single modification.

The structure of a co-crystal of peptide P43 with *^{Ec}*SC was solved at 1.6 Å. A T-shape p-stacking interaction between the phenyl group at the N-terminus of the peptide and the Phe278 was observed, in line with the previous observation with the fluorocinnamoyl derivative (peptide P18). Similarly, Arg365 also interacts with the aromatic ring through a cation-π interaction. At the other end of the peptide chain and despite the introduction of a Pro residue at position 5, in place of a Gly, the peptide binds to the SC with very close geometry of the Pol IV peptide (PDB 1OK7).

**Table 5. Structures, dissociation constants and thermodynamic parameters determined by ITC for the peptides combining modifications at different positions.**

| | | | | | | | |
|---|---|---|---|---|---|---|---|
| 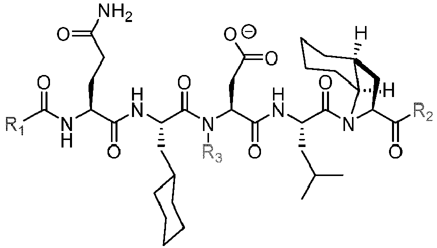 | | | | | | | |

| Compd | R₁ | R₂ | R₃ | *K*_{d} (nM) | ΔG (kcal mol⁻¹) | *kₒₙ* (M⁻¹ s⁻¹) | *k_{off}* (s) |
|---|---|---|---|---|---|---|---|
| **P47*** | CH₃ | 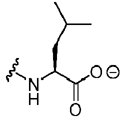 | H | 57 (± 20) | -9.9 (± 0.2) | 7,1 (±1.9) 10⁴ | 3,9 (±1.1) 10⁻³ |
| **P48** | CH₃ | 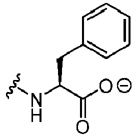 | H | 120 (± 57) | -9.5 (± 0.3) | 5.5 (±2.0) 10⁴ | 7.12 (±5.5) 10⁻³ |
| **P49** | 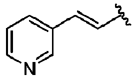 | 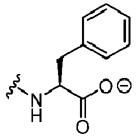 | Me | 118 (± 23) | -9.5 (± 0.1) | 4,7 (±0.3) 10⁴ | 5,57 (±0.8) 10⁻³ |
| **P50** | CH₃ | 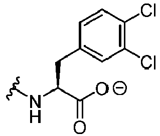 | H | 39 (± 11) | -10.1 (± 0.2) | 6.0 (±2.4) 10⁴ | 2.33 (±1.1) 10⁻³ |

| | | | | | | | |
|---|---|---|---|---|---|---|---|
| * The X-ray structure was obtained from protein-ligand co-crystal. | | | | | | | |

The proline residue in peptide P32 (K_{d} 182 nM) was thus replaced by the much larger octahydroindole-2-carboxylic acid (Oic) (peptide P47, Table 5). With a K_{d} value of 57 nM, this single amino acid substitution leads to a 3-fold increase in affinity compared to peptide P32 and to a remarkable 11-fold affinity enhancement in comparison to the Gly containing sequence derived from Pol IV (peptide P27). The X-ray-structure of peptide P47 bound to *^{Ec}*SC was solved with a resolution of 1.87 Å. It reveals that the groove is fully occupied by the octahydroindole moiety. Alternatively, this modification does not change much the thermodynamics of the interaction but reduces the *k_{off}* 6-fold as compared to peptide P42 (Tables 4 and 5). Several other derivatives bearing an Oic residue at that position were investigated. Based on the results showing that the replacement of the terminal Leu by a Phe in the hexamer series improves the affinity (compare peptides **P42 and P45**), the inventors thus prepared peptide P48 having both an Oic and Phe termination. However, the K_{d} value was not further improved. A very similar outcome was observed when combining Oic, the N-methylated aspartic residue and a pyridinyl acrylamide at the N-terminal position (peptide P49, to be compared to peptide P46). In contrast, the incorporation of the 3,4-dichlorinated phenylalanine at the C-terminal position further enhanced the affinity to give the best peptide ligand with a K_{d} of 39 nM (peptide P50, to be compared to P49).

## Claims

1. A compound of formula (I): 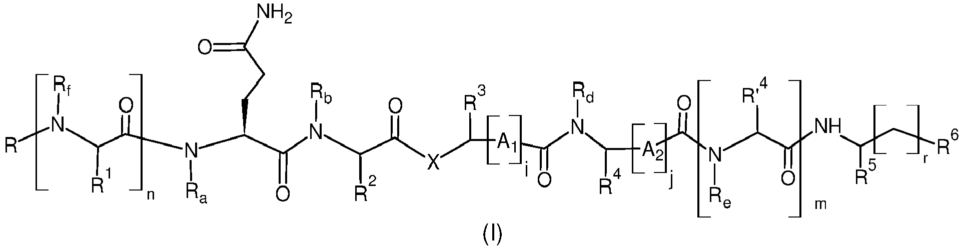 wherein:
- n is 0 or 1;
- m is 0 or 1;
- i is 0 or 1;
- j is 0 or 1;
- r is 0, 1 or 2;
- R is selected in the group consisting of:
* a (C₁-C₁₂)alkyl group optionally substituted by a (C₆-C₁₀)aryl group, by a heteroaryl group or by a heterocycloalkyl group,
* a (C₂-C₁₂)alkenyl group optionally substituted by a (C₆-C₁₀)aryl group,
* a (C₃-C₆)cycloalkyl group,
* a (C₆-C₁₀)aryl group optionally substituted by a (C₁-C₄)alkyl,
* a group having the following formula (II): 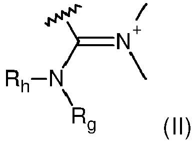 R_{g} and Rₕ representing independently from each other a (C₁-C₆)alkyl group or forming together with the nitrogen atom carrying them a heterocycloalkyl group,
* a group -C(=O)-R', R' being selected in the group consisting of:
• a (C₁-C₁₂)alkyl group optionally substituted by an optionally substituted (C₆-C₁₀)aryl group,
• a (C₂-C₁₂)alkenyl group optionally substituted by an optionally substituted C₆₋₁₀-aryl group or by a heteroaryl group,
• a -NH-optionally substituted (C₆-C₁₀)aryl group,
• a (C₃-C₆)cycloalkyl group,
• a (C₆-C₁₀)aryl group optionally substituted by a (C₁-C₄)alkyl,
* a group having the following formula (II-1): 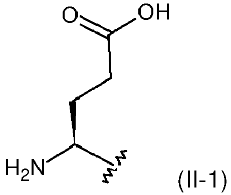
- X is O or a group N-R_{C};
- Rₐ, R_{b}, R_{c}, R_{d}, Rₑ, and R_{f} are, independently from each other, selected from the group consisting of: H, -CH₂-CH=CH₂, (C₁-C₆)alkyl groups, (C₆-C₁₀)aryl groups, and aralkyl groups;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
* q is 1, 2, 3 or 4,
* R^{7a} is a hydrogen atom, a (C₁-C₈)alkyl group, or an aralkyl group;
* R^{7b} is a hydrogen atom, a (C₁-C₈)alkyl group, or an aralkyl group,
* R⁸ is a hydrogen atom, a (C₁-C₈)alkyl group, or an aralkyl group, and
* R⁹ is H or COR¹¹, R¹¹ being H or a (C₁-C₈)alkyl group;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, and R'¹² being selected from the group consisting of: H, (C₁-C₈)alkyl group, and -C(=O)-(C₁-C₈)alkyl group;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)ₛ-, s being 1, 2, 3 or 4;
- R'⁴ is H, a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
or R'⁴ forms a heterocycloalkyl group with Rₑ, the carbon atom carrying it and the nitrogen atom carrying Rₑ, said heterocycloalkyl group being optionally substituted with at least one hydroxyl group, amino group, or sulfur atom;
- R⁵ is selected in the group consisting of:
* a -(CH₂)-(C₃-C₆)cycloalkyl group;
* a -(CH₂-CH₂)-(C₃-C₆)cycloalkyl group;
* a -(CH₂)-(C₆-C₁₀)aryl group optionally substituted by at least one halogen, (C₁-C₂)alkyl group and/or (C₁-C₂)alkoxy group;
* a -(CH₂-CH₂)-(C₆-C₁₀)aryl group optionally substituted by at least one halogen, (C₁-C₂)alkyl group and/or (C₁-C₂)alkoxy group;
* a -(CH₂)-(C₅-C₁₀)heteroaryl group optionally substituted by at least one halogen and/or (C₁-C₂)alkyl group;
* a -(CH₂-CH₂)-(C₅-C₁₀)heteroaryl group optionally substituted by at least one halogen and/or (C₁-C₂)alkyl group; and
* a (C₁-C₈)alkyl group or a (C₁-C₄)alkyl group optionally substituted by at least one halogen;
- R⁶ is H, -CO₂H, -CO₂R¹⁰, -CO-NH₂, -CO-NHR¹⁰, -OR¹⁰, -OH or NH₂ when r is 0 or 1 or 2, -NH-CO-NHR¹⁰ when r is 1 or 2; wherein
* R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group;
the following compounds being excluded:

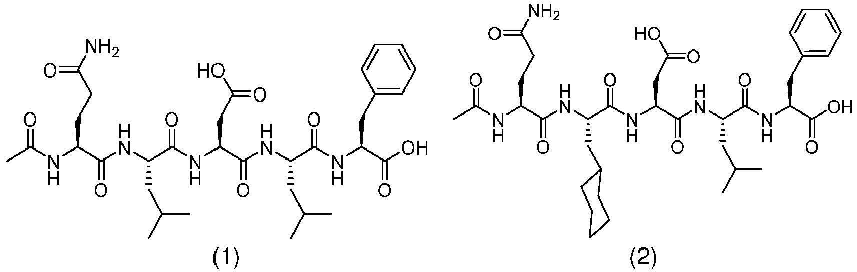

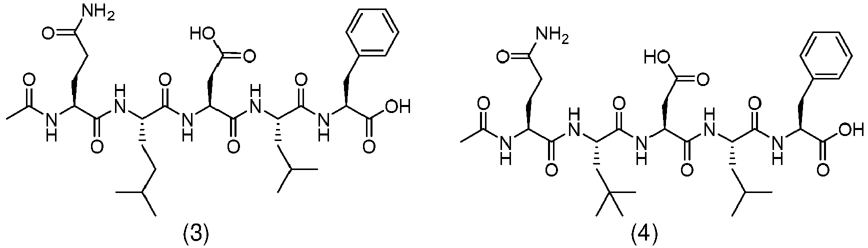

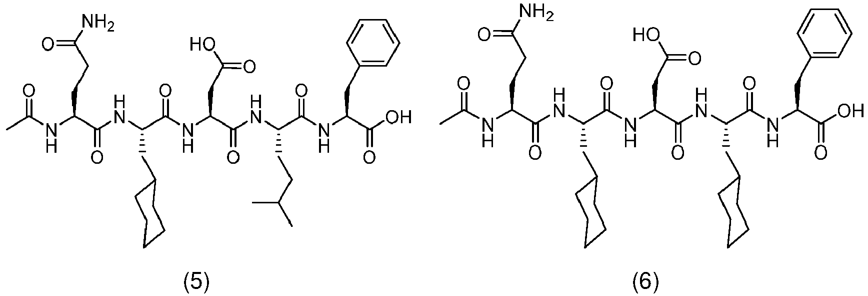

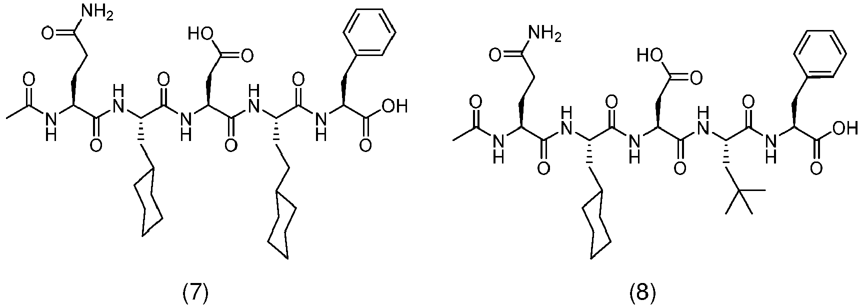

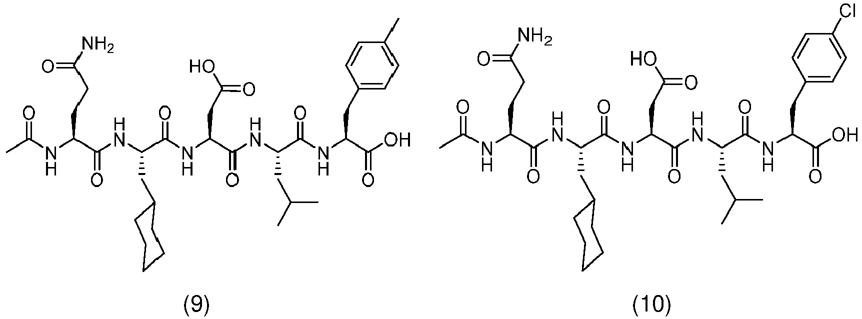

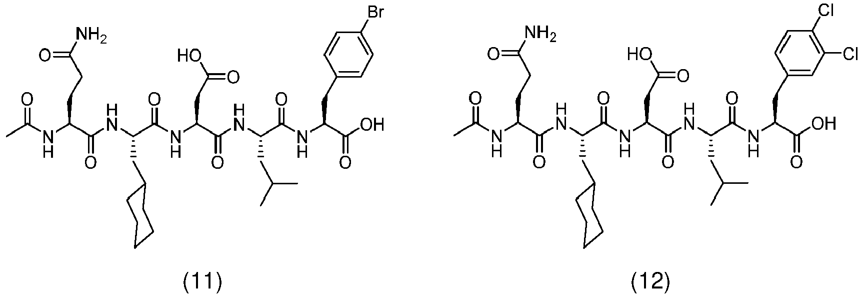

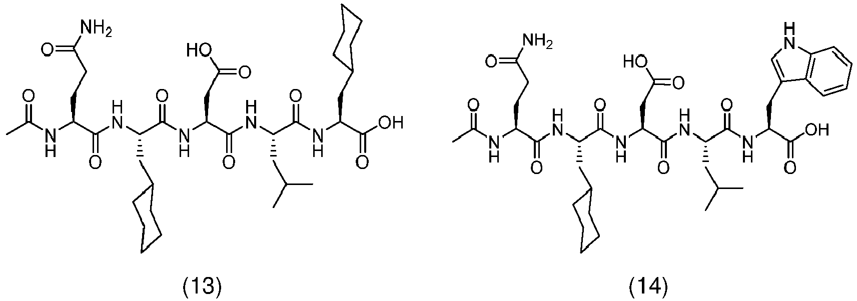

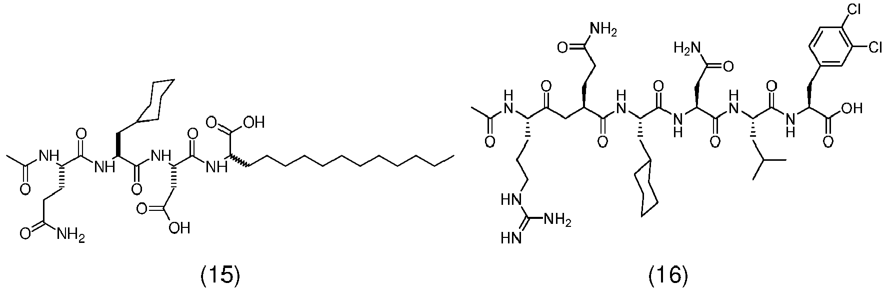

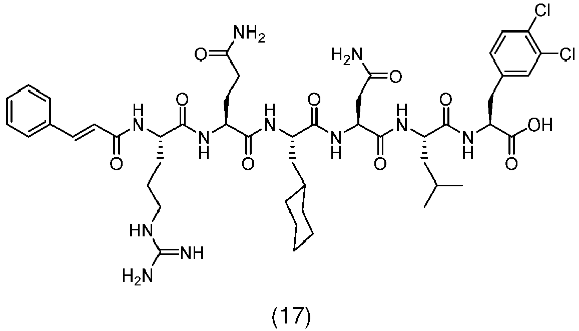

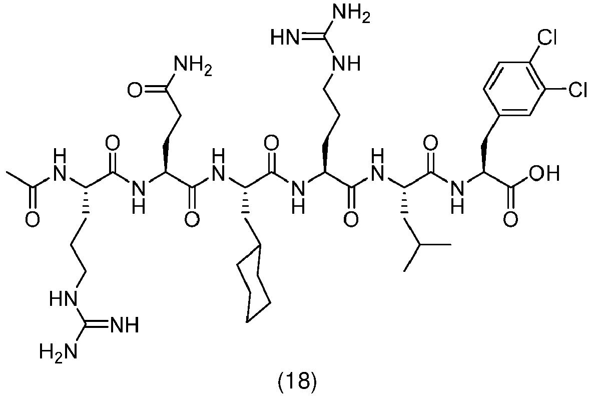

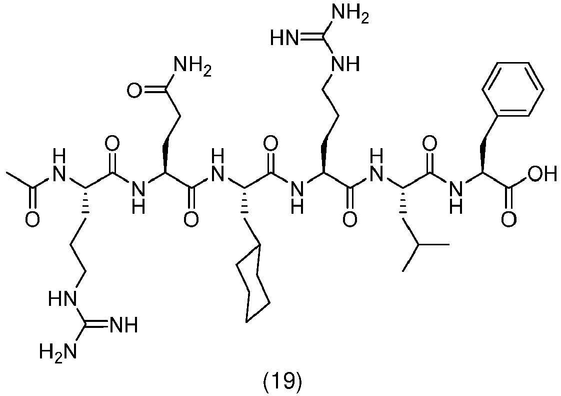

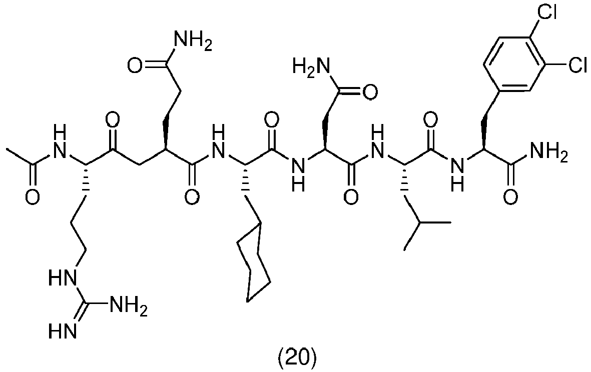

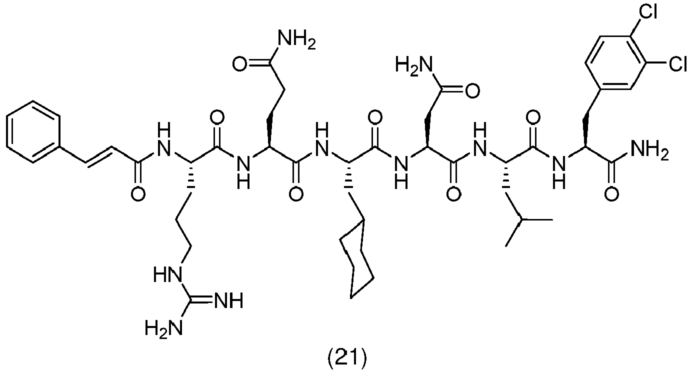

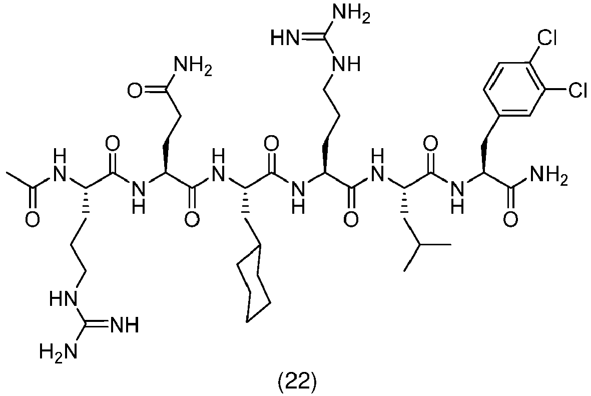

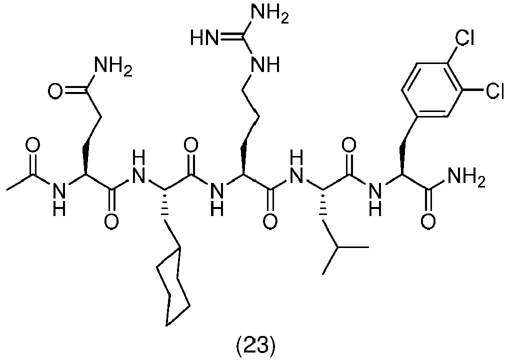

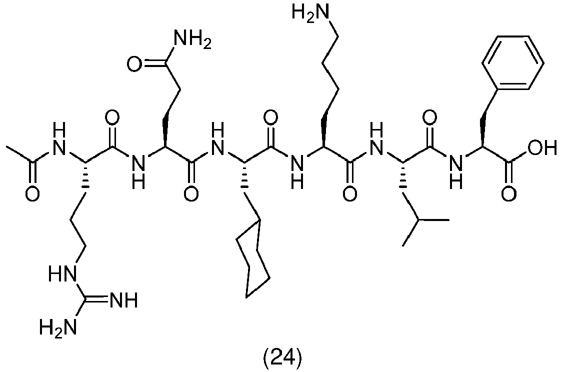

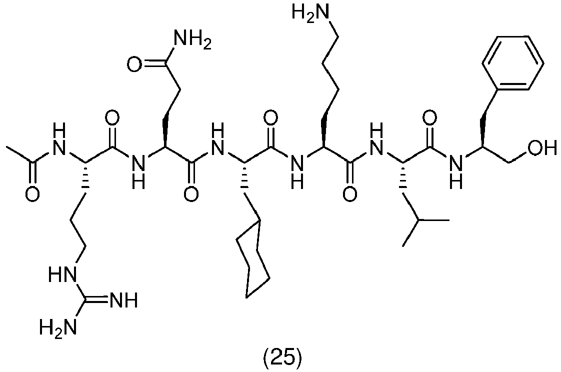

2. The compound of claim 1, wherein m=0 and at least one of Rₐ, R_{b}, R_{c}, R_{d}, Rₑ, and R_{f} is not H.

3. The compound of claim 1, wherein m=1.

4. The compound of the claim 1, wherein Rₐ, R_{b}, R_{d}, Rₑ, and R_{f} are H and R_{c} is Me.

5. The compound of claim 1, having the following formula (III): 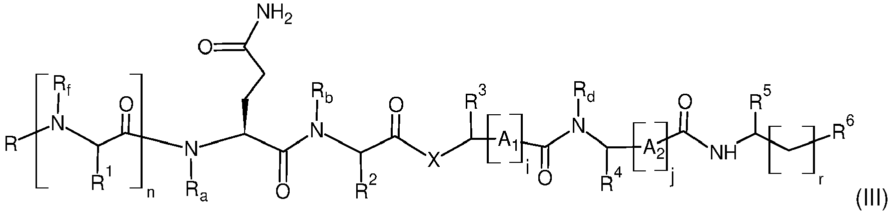 wherein:
- n is 0 or 1;
- i is 0 or 1;
- j is 0 or 1;
- r is 0, 1 or 2;
- R is selected in the group consisting of:
* a (C₁-C₁₂)alkyl group optionally substituted by a (C₆-C₁₀)aryl group, by a heteroaryl group or by a heterocycloalkyl group,
* a (C₂-C₁₂)alkenyl group optionally substituted by a C₆₋₁₀-aryl group,
* a (C₃-C₆)cycloalkyl group,
* a (C₆-C₁₀)aryl group optionally substituted by a C₁₋₄-alkyl,
* a group having the following formula (II): 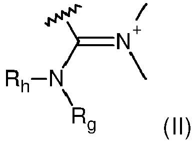 R_{g} and Rₕ representing independently from each other a (C₁-C₆)alkyl group or forming together with the nitrogen atom carrying them a heterocycloalkyl group,
* a group -C(=O)-R', R' being selected in the group consisting of:
• a (C₁-C₁₂)alkyl group optionally substituted by an optionally substituted (C₆-C₁₀)aryl group,
• a (C₂-C₁₂)alkenyl group optionally substituted by an optionally substituted C₆₋₁₀-aryl group or by a heteroaryl group,
• a -NH-optionally substituted (C₆-C₁₀)aryl group,
• a (C₃-C₆)cycloalkyl group,
• a (C₆-C₁₀)aryl group optionally substituted by a C₁₋₄-alkyl,
* a group having the following formula (II-1): 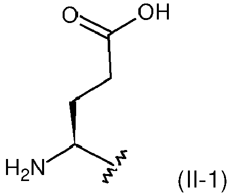
- X is O or a group N-R_{c};
- Rₐ, R_{b}, R_{c}, R_{d}, and R_{f} are, independently from each other, selected from the group consisting of: H, -CH₂-CH=CH₂, (C₁-C₆)alkyl groups, (C₆-C₁₀)aryl groups, and aralkyl groups, at least one of Rₐ, R_{b}, R_{c}, R_{d}, and R_{f} being not H;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
* q is 1, 2, 3 or 4,
* R^{7a} is a hydrogen atom, a (C₁-C₈)alkyl group, or an aralkyl group;
* R^{7b} is a hydrogen atom, a (C₁-C₈)alkyl group, or an aralkyl group,
* R⁸ is a hydrogen atom, a (C₁-C₈)alkyl group, or an aralkyl group, and
* R⁹ is H or COR¹¹, R¹¹ being H or a (C₁-C₈)alkyl group;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, and R'¹² being selected from the group consisting of: H, (C₁-C₈)alkyl group, and -C(=O)-(C₁-C₈)alkyl group;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)ₛ-, s being 1, 2, 3 or 4;
- R⁵ is selected in the group consisting of:
* a -(CH₂)-(C₃-C₆)cycloalkyl group;
* a -(CH₂-CH₂)-(C₃-C₆)cycloalkyl group;
* a -(CH₂)-(C₆-C₁₀)aryl group optionally substituted by at least one halogen, (C₁-C₂)alkyl group and/or (C₁-C₂)alkoxy group;
* a -(CH₂-CH₂)-(C₆-C₁₀)aryl group optionally substituted by at least one halogen, (C₁-C₂)alkyl group and/or (C₁-C₂)alkoxy group;
* a -(CH₂)-(C₅-C₁₀)heteroaryl group optionally substituted by at least one halogen and/or (C₁-C₂)alkyl group; and
* a -(CH₂-CH₂)-(C₅-C₁₀)heteroaryl group optionally substituted by at least one halogen and/or (C₁-C₂)alkyl group;
* a (C₁-C₈)alkyl group or a (C₁-C₄)alkyl group optionally substituted by at least one halogen;
- R⁶ is H, -CO₂H, -CO₂R¹⁰, -CO-NH₂, -CO-NHR¹⁰, -OR¹⁰, -OH or NH₂ when r is 0, 1 or 2, -NH-CO-NHR¹⁰ when r is 1 or 2; wherein
* R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group.

6. A compound of formula (IV): 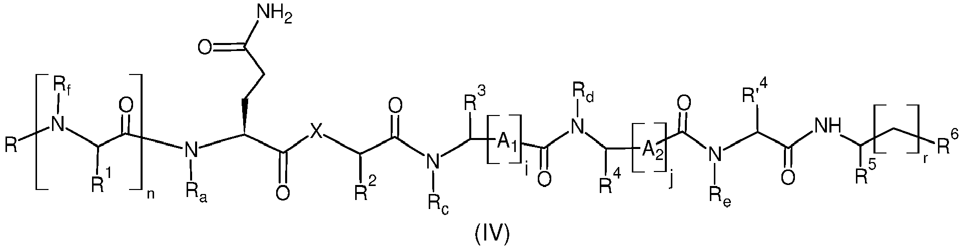 wherein:
- n is 0 or 1;
- i is 0 or 1;
- j is 0 or 1;
- r is 0, 1 or 2;
- R is selected in the group consisting of:
* a (C₁-C₁₂)alkyl group optionally substituted by a (C₆-C₁₀)aryl group, by a heteroaryl group or by a heterocycloalkyl group,
* a (C₂-C₁₂)alkenyl group optionally substituted by a (C₆-C₁₀)aryl group,
* a (C₃-C₆)cycloalkyl group,
* a (C₆-C₁₀)aryl group optionally substituted by a (C₁-C₄)alkyl,
* a group having the following formula (II): 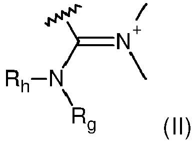 R_{g} and Rₕ representing independently from each other a (C₁-C₆)alkyl group or forming together with the nitrogen atom carrying them a heterocycloalkyl group,
* a group -C(=O)-R', R' being selected in the group consisting of:
• a (C₁-C₁₂)alkyl group optionally substituted by an optionally substituted (C₆-C₁₀)aryl group,
• a (C₂-C₁₂)alkenyl group optionally substituted by an optionally substituted C₆₋₁₀-aryl group or by a heteroaryl group,
• a -NH-optionally substituted (C₆-C₁₀)aryl group,
• a (C₃-C₆)cycloalkyl group,
• a (C₆-C₁₀)aryl group optionally substituted by a (C₁-C₄)alkyl,
* a group having the following formula (II-1): 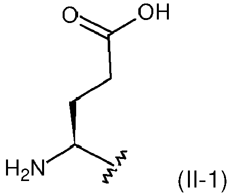
- X is O or a group N-R_{C};
- Rₐ, R_{b}, R_{c}, R_{d}, Rₑ, and R_{f} are, independently from each other, selected from the group consisting of: H, -CH₂-CH=CH₂, (C₁-C₆)alkyl groups, (C₆-C₁₀)aryl groups, and aralkyl groups;
- R¹ is H or the side chain of arginine;
- R² is a (C₁-C₆)alkyl group or a -CH₂-(C₃-C₆)cycloalkyl group optionally substituted by at least one halogen and/or by at least one group selected amongst -NH₂, -NH-CO-R'^{a}, -CO₂H, -NHR'^{a} and -NR'^{a}R'^{b}, wherein R'^{a} and R'^{b} are independently a (C₁-C₄)alkyl group;
or R² forms a heterocycloalkyl group with R_{b}, the carbon atom carrying it and the nitrogen atom carrying R_{b};
- R³ is selected in the group consisting of: H, a (C₁-C₈)alkyl group, the side chain of arginine, lysine, threonine, benzylthreonine, or phenylalanine, -(CH₂)_{q}-CO₂R^{7a}, -(CH₂)_{q}-CO-NHR^{7b}, -CH₂OR⁸, and -(CH₂)_{q}NHR⁹, wherein
* q is 1, 2, 3 or 4,
* R^{7a} is a hydrogen atom, a (C₁-C₈)alkyl group, or an aralkyl group;
* R^{7b} is a hydrogen atom, a (C₁-C₈)alkyl group, or an aralkyl group,
* R⁸ is a hydrogen atom, a (C₁-C₈)alkyl group, or an aralkyl group, and
* R⁹ is H or COR¹¹, R¹¹ being H or a (C₁-C₈)alkyl group;
- A₁ is a radical selected in the group consisting of: -(CH₂)ₚ- and -(CH₂)ₚNH-, p being 1, 2, 3 or 4; or A₁ being a radical -CH(NR¹²R'¹²)-, R¹² being a (C₁-C₈)alkyl group, and R'¹² being selected from the group consisting of: H, (C₁-C₈)alkyl group, and -C(=O)-(C₁-C₈)alkyl group;
- R⁴ is a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
- A₂ is a radical -(CH₂)ₛ-, s being 1, 2, 3 or 4;
- R'⁴ is H, a (C₁-C₈)alkyl group optionally substituted by a (C₃-C₆)cycloalkyl group, a (C₃-C₆)cycloalkyl group, or a (C₁-C₄)alkyl group optionally substituted by at least one halogen;
or R'⁴ forms a heterocycloalkyl group with Rₑ, the carbon atom carrying it and the nitrogen atom carrying Rₑ, said heterocycloalkyl group being optionally substituted with at least one hydroxyl group, amino group, or sulfur atom;
- R⁵ is selected in the group consisting of:
* a -(CH₂)-(C₃-C₆)cycloalkyl group;
* a -(CH₂-CH₂)-(C₃-C₆)cycloalkyl group;
* a -(CH₂)-(C₆-C₁₀)aryl group optionally substituted by at least one halogen, (C₁-C₂)alkyl group and/or (C₁-C₂)alkoxy group;
* a -(CH₂-CH₂)-(C₆-C₁₀)aryl group optionally substituted by at least one halogen, (C₁-C₂)alkyl group and/or (C₁-C₂)alkoxy group;
* a -(CH₂)-(C₅-C₁₀)heteroaryl group optionally substituted by at least one halogen and/or (C₁-C₂)alkyl group;
* a -(CH₂-CH₂)-(C₅-C₁₀)heteroaryl group optionally substituted by at least one halogen and/or (C₁-C₂)alkyl group; and
* a (C₁-C₈)alkyl group or a (C₁-C₄)alkyl group optionally substituted by at least one halogen;
- R⁶ is H, -CO₂H, -CO₂R¹⁰, -CO-NH₂, -CO-NHR¹⁰, -OR¹⁰, -OH or NH₂ when r is 0 or 1 or 2, -NH-CO-NHR¹⁰ when r is 1 or 2; wherein
* R¹⁰ is a (C₁-C₈)alkyl group optionally substituted by a (C₆-C₁₀)aryl group; a (C₃-C₆)cycloalkyl group; a (C₆-C₁₀)aryl group optionally substituted by a halogen, a (C₁-C₂)alkyl group and/or a (C₁-C₂)alkoxy group.

7. The compound of claim 5 or 6, wherein R is selected in the group consisting of:
* a (C₁-C₁₂)alkyl group optionally substituted by a (C₆-C₁₀)aryl group, by a heteroaryl group or by a heterocycloalkyl group,
* a group having the following formula (II): 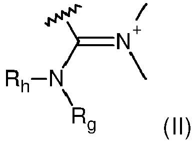 R_{g} and Rₕ representing independently from each other a (C₁-C₆)alkyl group or forming together with the nitrogen atom carrying them a heterocycloalkyl group,
* a group -C(=O)-R', R' being selected in the group consisting of:
• a (C₁-C₁₂)alkyl group optionally substituted by an optionally substituted (C₆-C₁₀)aryl group,
• a (C₂-C₁₂)alkenyl group optionally substituted by an optionally substituted C₆₋₁₀-aryl group or by a heteroaryl group,
• a -NH-optionally substituted (C₆-C₁₀)aryl group, and
* a group having the following formula (II-1):

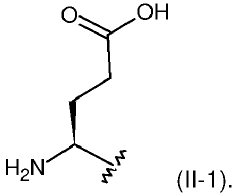

8. The compound of any one of claims 5 to 7, wherein R² is a -CH₂-(C₃-C₆)cycloalkyl group, preferably a -CH₂-cyclohexyl group.

9. The compound of any one of claims 5 to 8, wherein R³ is selected in the group consisting of: H, a (C₁-C₈)alkyl group, the side chain of arginine, lysine, threonine, benzylthreonine, or phenylalanine, -(CH₂)_{q}-CO₂R ^{7a}, -(CH₂)_{q}-CO-NHR^{7b}, - CH₂OR⁸, and -(CH₂)_{q}NH₂, wherein
* q is 1, 2, 3 or 4,
* R^{7a} is a hydrogen atom or a (C₁-C₈)alkyl group;
* R^{7b} is a hydrogen atom or a (C₁-C₈)alkyl group, and
* R⁸ is a hydrogen atom or a (C₁-C₈)alkyl group.

10. The compound of any one of claims 5 to 9, wherein R⁴ is a (C₁-C₈)alkyl group.

11. The compound of any one of claims 5 to 10, wherein R⁵ is a -(CH₂)-(C₆-C₁₀)aryl group optionally substituted by at least one halogen, (C₁-C₂)alkyl group and/or (C₁-C₂)alkoxy group, or a (C₁-C₈)alkyl group or a (C₁-C₄)alkyl group optionally substituted by at least one halogen.

12. The compound of any one of claims 5 to 11, wherein R⁶ is H, -CO₂H, - CO-NH₂, or -CO₂R¹⁰, R¹⁰ being a (C₁-C₈)alkyl group, preferably Me, or R⁶ is -OH or NH₂ when r is 1.

13. The compound of any one of claims 6 to 12, wherein R'⁴ is H or forms a heterocycloalkyl group with Rₑ, the carbon atom carrying it and the nitrogen atom carrying Rₑ, said heterocycloalkyl group being optionally substituted with at least one hydroxyl group.

14. The compound of any one of claims 1 to 13, having one of the following formulae:

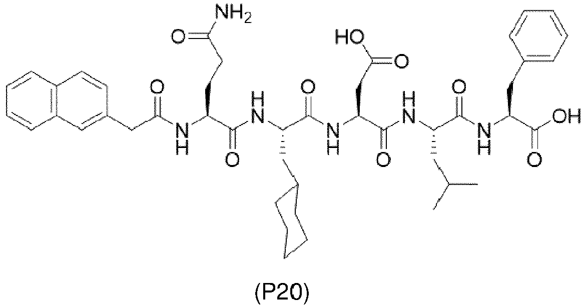

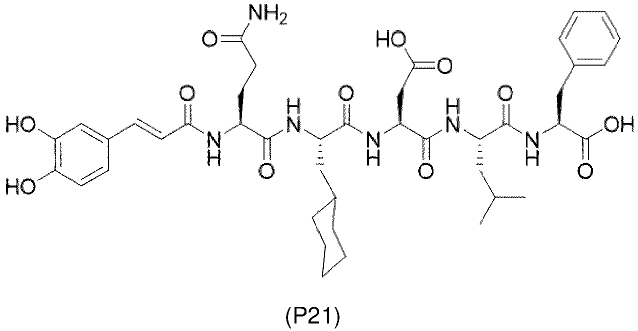

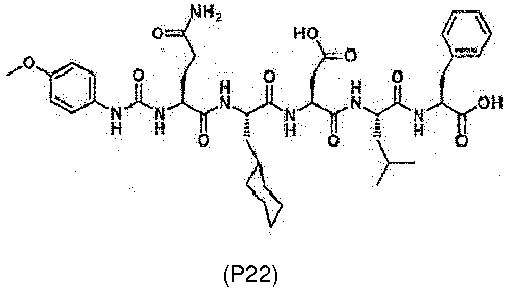

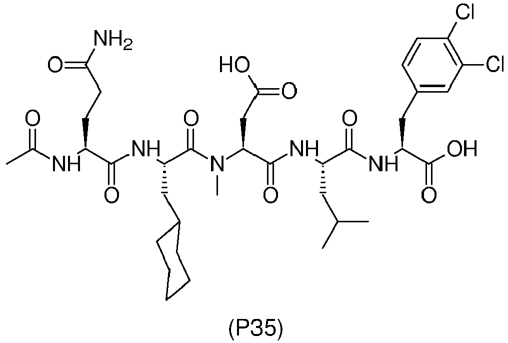

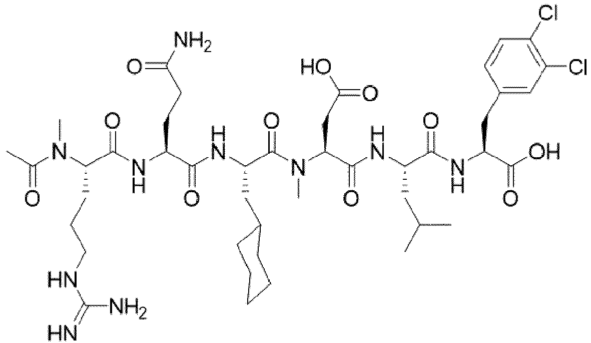

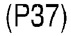

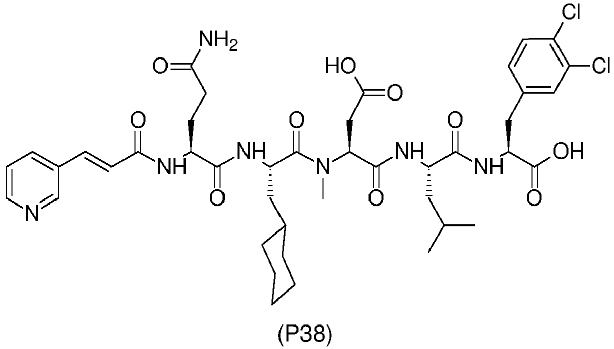

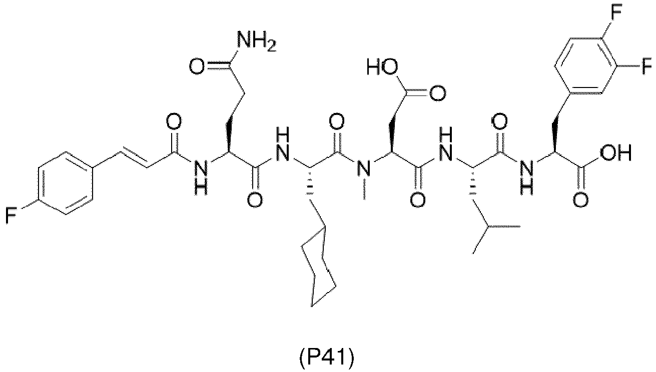

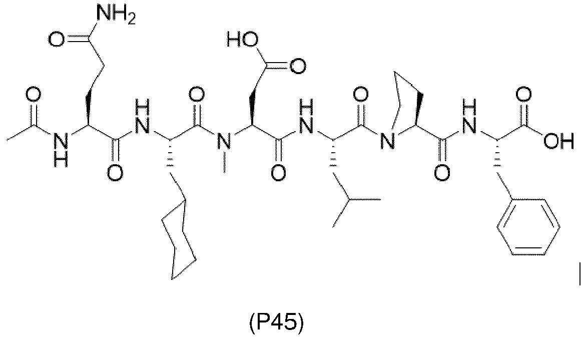

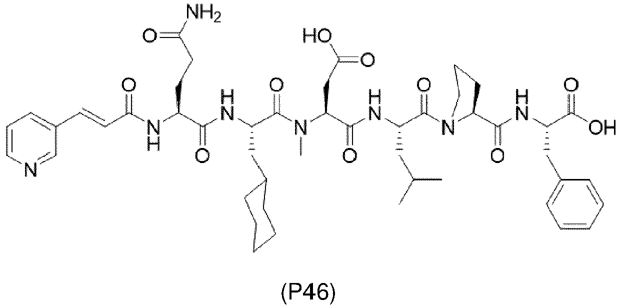

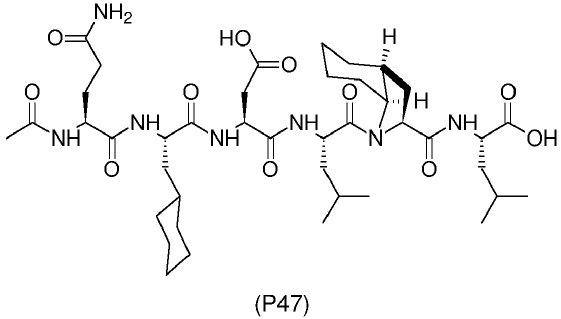

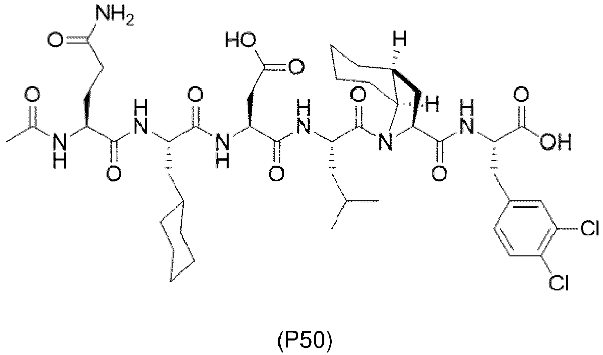

15. A medicament comprising a compound of any one of claims 1 to 14.

16. The compound of any one of claims 1 to 14 for use for the treatment of bacterial infections.
